# EUROPEAN PATENT APPLICATION

(11) **EP 3 690 053 A1**
(43) Date of publication of application: **05.08.2020**
(21) Application number: 18862737.6
(22) Date of filing: 20.09.2018
(51) Int. Cl.: C12N 15/85, C12N 15/10, C12N 15/113, C12N 9/22, C12N 9/10, C12N 9/64, A01K 67/027

(54) **FACTOR VIII OR FACTOR IX GENE KNOCKOUT RABBIT, METHOD FOR PREPARING SAME AND USE THEREOF**

(30) Priority: 28.09.2017 KR 20170126068
(71) Applicant: Green Cross Corporation, Yongin-si, Gyeonggi-do 16924 (KR); Mogam Institute for Biomedical Research, Yongin-si, Gyeonggi-do 16924 (KR)
(72) Inventor: KIM, So Ra, Yongin-si Gyeonggi-do 16924 (KR); JUNG, Myung Eun, Yongin-si Gyeonggi-do 16924 (KR); KIM, Min Jung, Yongin-si Gyeonggi-do 16924 (KR); JO, Seung Hyun, Yongin-si Gyeonggi-do 16924 (KR); HWANG, Sung Ho, Yongin-si Gyeonggi-do 16924 (KR); KWAK, Hee Chun, Yongin-si Gyeonggi-do 16924 (KR); LEE, Su Min, Yongin-si Gyeonggi-do 16924 (KR); NAM, Hyun Ja, Yongin-si Gyeonggi-do 16924 (KR)
(74) Representative: Mewburn Ellis LLP
(86) International application number: PCT/KR2018/011118
(87) International publication number: WO 2019/066378

(57) **Abstract**

The present invention relates to a factor VIII or factor IX gene knockout rabbit, a method for preparing the same and a use thereof and, more particularly, to a transgenic rabbit whose factor VIII or factor IX gene has been knocked out through the CRISPR/Cas9 system, a method for preparing the same and a use thereof. According to the present invention, in the transgenic rabbit, whose factor VIII and/or factor IX gene has been knocked out, the functions of factor VIII and/or factor IX, which are proteins that perform critical functions for the development of hemophilia, are inhibited, such that the transgenic rabbit is useful for the development of hemophilia treatments.

## Description

### [Technical Field]

The present invention relates to a factor VIII or factor IX gene knockout rabbit, a method of producing the same, and the use thereof. More specifically, the present invention relates to a transgenic rabbit having a factor VIII or factor IX gene knockout using a CRISPR/Cas9 system, a method of producing the same, and the use thereof.

### [Background Art]

Blood coagulation is a complicated and essential process that occurs in response to blood vessel damage. This is carried out by the formation of a thrombus that stops bleeding and begins the repair of damaged blood vessels; the damaged site is covered by fibrin and platelets including the thrombus. The process begins almost immediately after damage.

The coagulation process involves two types of ingredients: a cellular ingredient called "platelets" and a protein ingredient called a "coagulation factor". The platelets immediately form plugs at the site of injury, which is called "primary hemostasis". Secondary hemostasis refers to a phenomenon in which proteins in plasma, which appear simultaneously and are called coagulation factors or clotting factors, react through a complicated cascade process to form fibrin strands that strengthen platelet plugs.

The coagulation cascade of secondary hemostasis is divided into two pathways: the endogenous pathway, also called a "contact activation pathway", and the exogenous pathway, also called a "tissue factor pathway". Cofactors and modulators as well as a number of coagulation factors are involved to keep the process accurate.

For example, protein C is an essential factor in the main mechanism of coagulation regulation, called an "anticoagulant pathway". The active form of protein C (activating protein C) is a serine protease, which decomposes two factors of the coagulation cascade, namely factors Va and VIIIa, which are essential for the mass production of thrombin when associated with other cofactors (protein S). The destruction (decomposition) of these factors negatively regulates the amount of thrombin that is formed, leading to an anticoagulant effect. The protein is known to have pleiotropic biological activity, in particular, antithrombotic activity, anti-inflammatory activity, anti-apoptotic activity and pro-fibrinolytic activity.

Factor IX (hereinafter referred to as "FIX") is a serine protease that is essential for blood coagulation. Deficiency of this protein results in a bleeding disorder called "hemophilia B". During blood coagulation, activated FIX (FIXa) combines with its activated cofactor, factor VIIIa (hereinafter referred to as "FVIIIa"), which converts the specific substrate factor X (hereinafter referred to as "FX") to activated factor X (hereinafter referred to as "FXa"), which is an activated derivative thereof.

Factor X is another essential factor of the coagulation cascade. The activated form of FX (FXa) is the only serine protease that is capable of combining with its cofactor (coagulant factor Va) to activate prothrombin into thrombin. In addition, factor X, which has long been considered as a passive bystander, is an ingredient that is directly involved in a wide variety of cell types through activation of two major receptors thereof, namely protease-activated receptor-1 (PAR-1) and PAR-2. Recent findings have suggested that PAR-2 is an important mediator that regulates the interface between coagulation and disease progression and performs important functions in the point of factor X and in fibrotic diseases such as fibrosis, tissue remodeling and cancer (Borensztajn et al., Am J Pathol. 2008;172:309-20).

Among various blood coagulation factors responsible for hemostasis in the human body, deficiency of factor VIII is referred to as "hemophilia A", and deficiency of factor IX is referred to as "hemophilia B". Hemophilia A is the most common hereditary blood coagulation disease in the world, with the exception of von Willebrand disease, accounts for about 80 to 85% of all hemophilia, and has an incidence of 1 in 5,000 to 10,000 liveborn boys. Hemophilia B is more rare and is estimated to be about 1/5 of hemophilia A.

Normal subjects have factor VIII and factor IX activity of about 50 to 150% and exhibit decreased hemophilia A and B activity in blood tests. Depending on the activity of the coagulation factor, hemophilia is classified as severe (factor VIII or factor IX activity of 1% or less), moderate (the factor activity of 1 to 5%), mild (the factor activity of 5 to 30%), and subnormal (the factor activity of 30 to 50%) and the symptoms vary depending on the degree of severity. For example, in patients with severe hemophilia A, spontaneous bleeding may occur at any time without special trauma. Before the development of the therapeutic agent (factor VIII concentrate), the average life expectancy was only 25 years due to cerebral hemorrhage. Regarding severity degree distribution, the incidences of severe, moderate and mild patients of hemophilia A were about 70%, 15% and 15%, respectively, and the incidences of severe, moderate and mild patients of hemophilia B are about 50%, 30% and 20%, respectively. The incidence of bleeding is generally known to be once a week for severe patients, once a month for moderate patients, and once a year for mild patients. The joints and muscles are the most common bleeding sites. In particular, joint bleeding is particularly noteworthy between the ages of 15 and 25, and when bleeding is repeated, the patient suffers from arthrogryposis due to hemophilic arthropathy for an average of 50 years.

It is very important to predict the inhibitor development (antibody production) response when treating hemophilia using medicine. The incidences of inhibitor development response are about 30% and about 3% in hemophilia A patients and hemophilia B patients, respectively, (Kessler CM, Hematology. Am. Soc. Hematol. Educ. Program. 2005:429-35). Antibodies to hemophilia drugs are produced most frequently within 50 days after exposure to drugs and are typically classified into an antibody-producing group exhibiting a Bethesda unit (BU) of 0.6 or more and a hyperantibody-producing group exhibiting 5 BU or more (Kasper CK et al., Thromb. Diath. Haemorrh. 1975;34(2):612; Verbruggen B et al., Thromb. Haemost. 1995;73(2):247-51; Viel KR et al., N. Engl. J. Med. 2009;360(16):1618-27; Kemton CL et al., Blood. 2009;113(1):11-7). It is very difficult to treat patients having an antibody-producing reaction, and particularly, they often suffer from joint complications due to frequent bleeding (Park YS, J Korean Med Assoc 2009; 52(12):1201-6). Alternative therapies include administration of bypassing factors such as activating prothrombin complexes and factor VII recombinant agents, but it may be more preferable to remove antibodies from the group having antibody production reaction through immunotolerance (Kemton CL et al., Blood. 2009;113(1):11-7; Park YS, J. Korean Med. Assoc. 2009; 52(12):1201-6). The success rate of immunotolerance varies from 30 to 80%, and after immunotolerance, patients can continue to use the constant factor VIII or factor IX agents (Park YS, J. Korean Med. Assoc. 2009; 52(12):1201-6).

Several studies have been conducted on the major predictors or causes of antibody production response for hemophilia treatment. Among the genetic predictors, defects of the F8 gene are known to be the greatest cause (Schwaab R et al., Thromb. Haemost. 1995 74 (6) :1402-6; Oldenburg J et al., Thromb. Haemost. 1997, 77(2):238-42) and single-nucleotide polymorphism (SNP) mutations disposed in genes such as MHC II, TNF-α, IL-10 and CTLA-4 are also considered to have significant statistical associations, but it is predicted that there are a number of genetic predictors that have not yet been identified (Hay CR et al., Thromb. Haemost. 1997 77(2):234-7; Oldenburg J et al., Thromb. Haemost. 1997;77(2):238-42; Astermark J et al., Blood. 2006a, 107(8):3167-72; Astermark J et al., Blood. 2006b, 108(12):3739-45; Astermark J et al., J Thromb. Haemost. 2007, 5(2):263-5) .

Meanwhile, since the CRISPR/Cas system, which is an immune system that protects microorganisms from viruses, was introduced into heterologous cells in 2012, use of the system for selective cutting of a target base sequence and for editing of the genome of a wide variety of cells from microorganisms to human cells has been shown to be possible, and thus the CRISPR/Cas system is expected to be utilized more efficiently and conveniently in biological improvement as a gene-editing technology (Jinek et al., Science, 337(6096): 816-821, 2012).

In the gene-editing technique, among CRISPR/Cas systems, the CRISPR/Cas9 system generates double-strand breaks (DSBs) on the target DNA by Cas9 and sgRNA constituting the CRISPR/Cas9 system, and the cell recognizes the DSBs as injury sites to induce non-homologous end joining (NHEJ) or typical DNA repair by homology-directed repair (HDR). During this process, normalization is possible by mutation or gene replacement, thus inducing genome editing using the same. The non-homologous end joining (NHEJ) mechanism includes arranging the DSBs produced by the action of the CRISPR/Cas system, followed by simple joining. During this process, a frameshift mutation is introduced so that a gene deletion can easily be induced. Meanwhile, homologous-directed repair (HDR) mechanisms can occur in the presence of fragments homologous to truncated regions, which can lead to normalization or deletion by gene substitution.

This CRISPR/Cas system is very advantageous for the production of transgenic animals because it can delete a target gene at a precise position. Efforts have been made to produce transgenic animals based on the CRISPR/Cas system for hemophilia research. Recently, FVIII/FIX knock-out mice obtained by applying the CRISPR/Cas system to NSG mice (Nod/Scid-Il2γ-/-) have been reported (Ching-Tzu Yen, et al., Thrombosis Journal, Vol. 14:22, 2016). Rabbits are promising animal models for biomedical research because rabbits are more similar to humans in physiology and anatomy than mice and incur lower maintenance costs and have shorter gestation periods than pigs or monkeys. However, no hemophilia rabbit model using a CRISPR/Cas system has been known to date.

Under this background, as a result of intensive efforts to produce a rabbit, from which factor VIII and/or factor IX is knocked out, the present inventors have found that rabbits, from which the factor VIII and/or factor IX is knocked out, can be produced using the CRISPR/Cas system including sgRNA capable of complementarily binding to the exon region of the factor VIII and/or factor IX, thus completing the present invention.

The above information disclosed in this Background section is provided only for enhancement of understanding of the background of the invention, and therefore it may include information that does not form the prior art that is already known in this country to a person of ordinary skill in the art.

### [Disclosure]

### [Technical Problem]

It is one object of the present invention to provide sgRNA including a targeting domain complementarily binding to exon 1 and exon 2 regions of each of factor VIII (FVIII) or factor IX (FIX), a vector including the same and a CRISPR/Cas9 system including the vector.

It is another object of the present invention to provide a transgenic rabbit from which factor VIII and/or factor IX is knocked out, produced using the CRISPR/Cas9 system, and a method of producing the same.

It is another object of the present invention to provide the use of the transgenic rabbit from which factor VIII and/or factor IX is knocked out as a model for hemophilia research.

### [Technical Solution]

In accordance with one aspect of the present invention, the above and other objects can be accomplished by the provision of sgRNA including a targeting domain complementarily binding to a part of exon regions of factor VIII (FVIII) or factor IX (FIX).

In another aspect of the present invention, provided are a polynucleotide encoding the sgRNA, a vector into which the polynucleotide is inserted, a CRISPR/Cas9 system including the vector, and a transgenic rabbit, from which factor VIII and/or factor IX is knocked out, produced using the CRISPR/Cas9 system.

In another aspect of the present invention, provided is a method of producing a transgenic rabbit from which factor VIII and/or factor IX is knocked out, including (a) transcribing the CRISPR/Cas9 system to produce sgRNA and Cas9 mRNA, (b) introducing the mRNA produced in step (a) into an embryo and culturing the embryo, and (c) transplanting the embryo obtained in step (b) to a surrogate mother to produce the transgenic rabbit.

In another aspect of the present invention, provided are cells, tissues and byproducts isolated from the transgenic rabbit from which factor VIII and/or factor IX is knocked out.

### [Description of Drawings]

FIG. 1 is a schematic diagram (A) showing the position of a factor VIII gene targeted by sgRNA prepared for producing a transgenic rabbit according to an embodiment of the present invention and a schematic diagram (B) showing the position of a factor IX gene targeted by the sgRNA prepared for producing a transgenic rabbit according to an embodiment of the present invention.
FIG. 2 shows (A) the sequence of an amplicon used in NGS-based sequencing analysis to detect knockout of factor VIII in the transgenic rabbit prepared according to the present invention and (B) the sequence of an amplicon used in NGS-based sequencing analysis to detect knockout of factor IX in the transgenic rabbit prepared in the present invention.
FIG. 3 shows (A) a result confirming that 4 bp of the factor VIII gene is deleted from the second factor VIII knockout rabbit (#2) produced according to the present invention and (B) a result confirming the deletion mutation of the factor VIII gene in the third factor VIII knockout rabbit (#3) produced according to the present invention.
FIG. 4 shows (A) a result confirming the deletion mutation of the factor IX gene in the sixth factor IX knockout rabbit (#6) produced according to the present invention, (B) a result confirming the deletion mutation of the factor IX gene in the seventh factor IX knockout rabbit (#7) produced according to the present invention, and (C) a result confirming the deletion mutation of the factor IX gene in the eighth factor IX knockout rabbit (#8) produced according to the present invention.
FIG. 5 shows (A) a result confirming the deletion mutation of the factor IX gene in the ninth factor IX knockout rabbit (#9) produced according to the present invention, and (B) a result confirming the deletion mutation of the factor IX gene in the eleventh factor IX knockout rabbit (#11) produced according to the present invention.
FIG. 6 shows (A) a result confirming the deletion mutation of the factor IX gene in the twelfth factor IX knockout rabbit (#12) produced according to the present invention, (B) a result confirming the deletion mutation of the factor IX gene in the thirteenth factor IX knockout rabbit (#13) produced according to the present invention, and (C) a result confirming the deletion mutation of the factor IX gene in the fifth factor IX knockout rabbit (#5) produced according to the present invention.

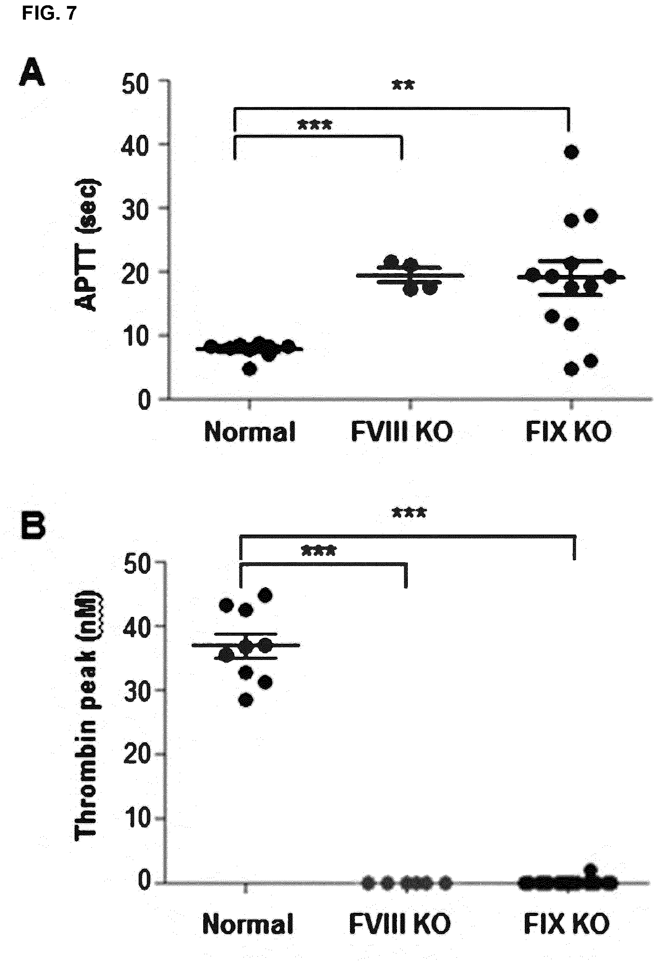

FIG. 7 is (A) a graph showing the results of APTT analysis of the transgenic rabbit from which factor VIII or factor IX is knocked out, wherein experiments are performed two or three times with plasma of four normal rabbits, two rabbits from which factor VIII is knocked out, and seven rabbits from which factor IX is knocked out and (B) a graph showing the results of TGA analysis conducted two or three times with plasma of nine normal rabbits, three rabbits from which factor VIII is knocked out, and eight rabbits from which factor IX is knocked out, wherein the results are indicated as mean ± s.e.m., statistical significance was determined using a two-tailed, unpaired t-test, ** represents p < 0.01, and *** represents p < 0.001.
FIG. 8 shows the breeding pedigree for the production of F1 and F2 generations.
FIG. 9 shows a result confirming the gene Indel mutation of female progeny obtained by crossing a male second factor VIII knockout rabbit (#2) produced according to the present invention with a normal female.
FIG. 10 shows a result confirming the gene deletion mutation of female progeny obtained by crossing a male sixth factor IX knockout rabbit (#6) produced according to the present invention with a normal female.
FIG. 11 shows a result confirming the gene Indel mutation of the F2 male obtained by crossing a female (X'X) F1-generation of the factor VIII knockout rabbit produced according to the present invention with a normal male.
FIG. 12 shows a result confirming the gene Indel mutation of the F2 male obtained by crossing a carrier female (X'X) F1-generation of the factor IX knockout rabbit produced according to the present invention with a normal male.
FIG. 13 is a schematic diagram illustrating the production of a rabbit claw bleeding model.
FIG. 14 shows the amount of hemoglobin in the blood measured from a hemolyzed sample.

### [Best Mode]

Unless defined otherwise, all technical and scientific terms used herein have the same meanings as appreciated by those skilled in the field to which the present invention pertains. In general, the nomenclature used herein is well-known in the art and is ordinarily used.

As used herein, the terms "nucleic acid" and "polynucleotide" refer to a deoxyribonucleotide or ribonucleotide polymer in a linear or cyclic three-dimensional form and in a single- or double-stranded form. For the objects of the present invention, these terms should not be construed as limiting the length of the polymer. These terms may encompass known analogues of natural nucleotides as well as nucleotides that are modified at base, sugar and/or phosphate moieties (e.g., phosphorothioate backbones). In general, analogues of certain nucleotides have the same base-pairing specificity; that is, analogues of A form a base pair with T.

As used herein, the term "nucleotide" refers to deoxyribonucleotide or ribonucleotide. The nucleotide may be a standard nucleotide (i.e., adenosine, guanosine, cytidine, thymidine and uridine) or a nucleotide analogue. The nucleotide analogue refer to a nucleotide having a modified purine or pyrimidine base or modified ribose moiety. The nucleotide analogue may be a naturally derived nucleotide (e.g., inosine) or an artificially derived nucleotide. Non-limiting examples of modifications of sugar or base moieties of the nucleotide include the addition (or removal) of acetyl groups, amino groups, carboxyl groups, carboxymethyl groups, hydroxyl groups, methyl groups, phosphoryl groups and thiol groups as well as substitution of carbon and nitrogen atoms of the base with other atoms (e.g., 7-deaza purine). Nucleotide analogues also include dideoxy nucleotides, 2'-O-methyl nucleotides, locked nucleic acids (LNA), peptide nucleic acids (PNA) and morpholino.

As used herein, the term "sgRNA" refers to a first region that complementarily binds to a target region in a guide RNA that guides a Cas protein to a target region in a CRISPR/Cas system.

In the present invention, the guide RNA interacts with the Cas protein to direct the Cas protein to a specific target site, wherein the 5' end of the guide RNA forms a base pair with a particular protospacer sequence within the chromosomal sequence.

Each guide RNA includes three regions: the first region at the 5' end, which is complementary to the target site within the chromosome sequence, the second inner region, which forms a stem loop structure, and the third 3' region, which remains essentially as a single strand. domain. The first regions of respective guide RNAs are different such that each guide RNA directs the fusion protein to a specific target site. The second and third regions of each guide RNA may be the same in all guide RNAs.

The first region of the guide RNA is complementary to the sequence (i.e., the protospacer sequence) at the target site within the chromosomal sequence, such that the first region of the guide RNA is capable of forming a base pair with the target site. In various embodiments, the first region of the guide RNA may include about 10 nucleotides or more than about 25 nucleotides. For example, the region of base pairing between the first region of the guide RNA and the target site in the chromosomal sequence may be about 10, 11, 12, 13, 14, 15, 16, 17, 18, 19, 20, 22, 23, 24 or 25 nucleotides, or more than 25 nucleotides in length. In an exemplary embodiment, the first region of the guide RNA is about 19, 20 or 21 nucleotides in length.

The guide RNA also includes the second region that forms a secondary structure. In some embodiments, the secondary structure includes a stem (or hairpin) and a loop. The lengths of the loop and stem may vary. For example, the loop may vary in the range of about 3 to about 10 nucleotides in length, and the stem may vary in the range of about 6 to about 20 base pairs in length. The stem may include one or more protrusions of 1 to about 10 nucleotides. Thus, the overall length of the second region may vary from about 16 to about 60 nucleotides in length. In an exemplary embodiment, the loop is about 4 nucleotides in length, and the stem includes about 12 base pairs.

The guide RNA also includes the third region at the 3' end that remains essentially as a single strand. Thus, the third region has no complementarity to any chromosomal sequence in the cell of interest, and no complementarity to the rest of the guide RNA. The length of the third region may vary. Generally, the third region is more than about 4 nucleotides in length. For example, the length of the third region may vary from about 5 to about 60 nucleotides in length.

The total length of the second and third regions (also called "universal or skeletal regions") of the guide RNA may vary in length from about 30 to about 120 nucleotides. In one aspect, the total length of the second and third regions of the guide RNA varies from about 70 to about 100 nucleotides in length.

In the present invention, whether or not a rabbit, from which factor VIII and/or factor IX is knocked out, can be produced using the CRISPR/Cas system was determined.

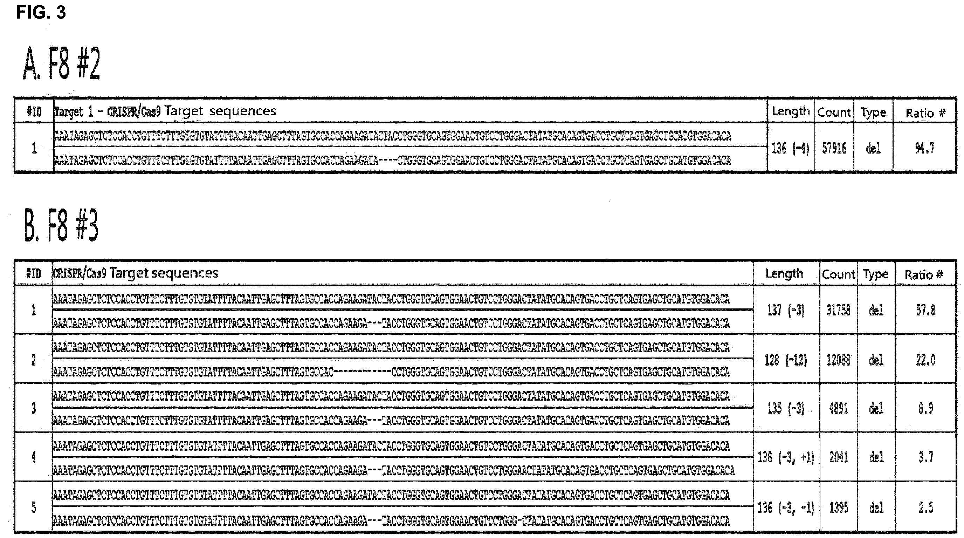

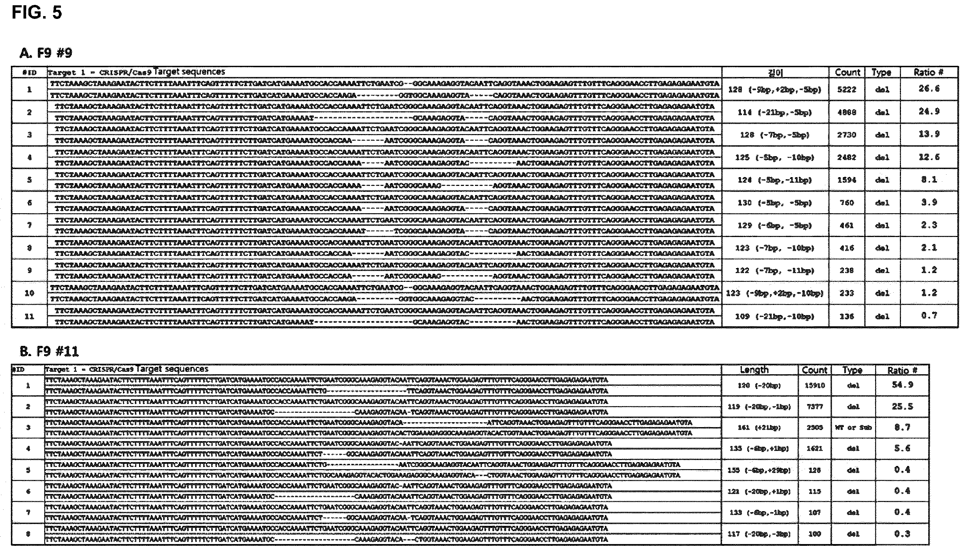

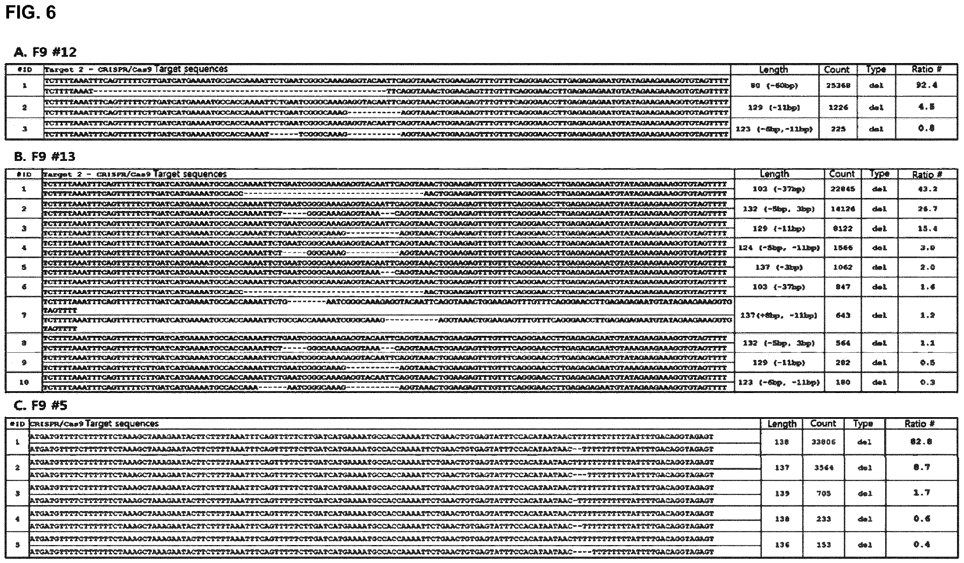

That is, in one embodiment of the present invention, an sgRNA including a targeting domain that complementarily binds to a part of the exon region of factor VIII (FVIII) or factor IX (FIX) is produced, the CRISPR/Cas system including the same is transcribed, and the sgRNA is injected into a rabbit embryo, cultured and transplanted into a surrogate mother to product a rabbit. The result showed that deletion mutations occur in the exon region of factor VIII or factor IX of the produced rabbit (FIGS. 3 to 7).

Thus, in one aspect, the present invention relates to an sgRNA including a targeting domain that complementarily binds to a part of the exon region of factor VIII (FVIII) or factor IX (FIX).

In the present invention, the exon region of the factor VIII (FVIII) gene may be an exon region represented by the base sequence of SEQ ID NO: 1, but is not limited thereto.

In the present invention, the exon region of the factor IX (FIX) gene may be an exon region represented by the base sequence of SEQ ID NO: 2, but is not limited thereto.

The present invention also relates to a polynucleotide encoding the sgRNA.

In the present invention, the polynucleotide may be represented by the base sequence of any one of SEQ ID NOS: 3 to 6, but is not limited thereto.

In the present invention, the polynucleotide encoding the sgRNA is generally operably linked to at least one transcriptional control sequence for expression of the sgRNA in the cell or embryo of interest. For example, DNA encoding sgRNA may be operably linked to a promoter sequence recognized by RNA polymerase III (Pol III). Examples of suitable Pol III promoters include, but are not limited to, mammalian U6, U3, H1 and 7SL RNA promoters.

The present invention also relates to a vector into which the polynucleotide is inserted.

DNA molecules encoding sgRNAs may be linear or cyclic. In some embodiments, the DNA sequence encoding sgRNA can be a part of a vector. Suitable vectors include plasmid vectors, phagemids, cosmids, artificial/mini chromosomes, transposons and viral vectors. In an exemplary embodiment, the DNA encoding the Cas protein is present in a plasmid vector. Non-limiting examples of suitable plasmid vectors include pUC, pBR322, pET, pBluescript and variants thereof. Vectors may include additional expression control sequences (e.g., enhancer sequences, Kozak sequences, polyadenylation sequences, or transcription termination sequences), selectable marker sequences (e.g., antibiotic-resistant genes), origins of replication, and the like.

In specific embodiments wherein both the Cas protein and the sgRNA are introduced into the cell as DNA molecules, each may be a part of a separate molecule (e.g., one vector including the fusion-protein-coding sequence and the second vector including the sgRNA-coding sequence), or both may be a part of the same molecule (e.g., one vector including coding (and control) sequences for both the fusion protein and the guide RNA).

The present invention also relates to a CRISPR/Cas system including the vector.

In the present invention, the CRISPR/Cas system may be a type I, type II or type III system. Non-limiting examples of suitable CRISPR/Cas proteins include Cas3, Cas4, Cas5, Cas5e (or CasD), Cas6, Cas6e, Cas6f, Cas7, Cas8a1, Cas8a2, Cas8b, Cas8c, Cas9, Cas10, Cas10d, CasF, CasG, CasH, Csy1, Csy2, Csy3, Cse1 (or CasA), Cse2 (or CasB), Cse3 (or CasE), Cse4 (or CasC), Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csz1, Csx15, Csf1, Csf2, Csf3, Csf4, and Cu1966.

In the present invention, the CRISPR/Cas protein is derived from the Cas9 protein. The Cas9 protein is derived from *Streptococcus pyogenes, Streptococcus thermophilus, Streptococcus* species, *Nocardiopsis dassonvillei, Streptomyces pristinaespiralis, Streptomyces viridochromogenes, Streptosporangium roseum, Alicyclobacillus acidocaldarius, Bacillus pseudomycoides, Bacillus selenitireducens, EIXguobacterium sibiricum, Lactobacillus delbrueckii, Lactobacillus salivarius, Microscilla marina, Burkholderiales* bacterium, *Polaromonas naphthalenivorans, Polaromonas* species, *Crocosphaera watsonii, Cyanothece* species, *Microcystis aeruginosa, Synechococcus* species, *Acetohalobium arabaticum, Ammonifex degensii, Caldicellulosiruptor bescii, Candidatus Desulforudis, Clostridium botulinum, Clostridium difficile, Finegoldia magna, Natranaerobius thermophilus, Pelotomaculum thermopropionicum, Acidithiobacillus caldus, Acidithiobacillus ferrooIXdans, Allochromatium vinosum, Marinobacter* species, *Nitrosococcus halophilus, Nitrosococcus watsonii, Pseudoalteromonas haloplanktis, Ktedonobacter racemifer, Methanohalobium evestigatum, Anabaena variabilis, Nodularia spumigena, Nostoc* species, *Arthrospira maIXma, Arthrospira platensis, Arthrospira* species, *Lyngbya* species, *Microcoleus chthonoplastes, Oscillatoria* species, *Petrotoga mobilis, Thermosipho africanus,* or *Acaryochloris marina.*

Generally, CRISPR/Cas proteins include at least one RNA recognition and/or RNA-binding domain. The RNA recognition and/or RNA-binding domain interacts with the guide RNA. CRISPR/Cas proteins also include nuclease domains (i.e., DNAase or RNAase domains), DNA-binding domains, helicase domains, RNAase domains, protein-protein interaction domains, dimerization domains and other domains. The CRISPR/Cas-like protein may be a wild-type CRISPR/Cas protein, a modified CRISPR/Cas protein, or a fragment of a wild-type or modified CRISPR/Cas protein. CRISPR/Cas-like proteins may be modified to improve nucleic acid binding affinity and/or specificity, change enzymatic activity, and/or change other protein properties. For example, the nuclease (i.e., DNAase, RNAase) domain of the CRISPR/Cas-like protein may be modified, deleted or inactivated. Alternatively, CRISPR/Cas-like proteins may be truncated to remove domains that are not essential for the function of the fusion protein. CRISPR/Cas-like proteins may also be truncated or modified to optimize the activity of the effector domain of the fusion protein.

In some embodiments, the CRISPR/Cas-like protein may be derived from a wild-type Cas9 protein or a fragment thereof. In other embodiments, the CRISPR/Cas-like protein may be derived from a modified Cas9 protein. For example, the amino acid sequence of a Cas9 protein may be modified to change one or more properties of the protein (e.g., nuclease activity, affinity and stability). Alternatively, domains of the Cas9 protein that are not involved in RNA-induced cleavage may be removed from the protein, so the modified Cas9 protein is smaller than the wild-type Cas9 protein.

In general, the Cas9 protein includes at least two nuclease (i.e., DNAase) domains. For example, the Cas9 protein may include a RuvC-like nuclease domain and an HNH-like nuclease domain. The RuvC and HNH domains work together to cut single strands and to thus produce double-stranded breaks in DNA (Jinek et al., Science, 337: 816-821). In some embodiments, the Cas9-derived protein may be modified to include only one functional nuclease domain (either the RuvC-like domain or HNH-like nuclease domain). For example, the Cas9-derived protein may be modified such that one of the nuclease domains is deleted or mutated and thus has no function any more (i.e., such that nuclease activity is not exhibited) . In some embodiments, in which one of the nuclease domains is inactive, the Cas9-derived protein may introduce a gap into the double-stranded nucleic acid (such a protein is called a "nickase"), but does not cleave the double-stranded DNA. For example, conversion from aspartate to alanine (D10A) in the RuvC-like domain converts Cas9-derived proteins into ligase. Similarly, conversion from histidine to alanine (H840A or H839A) in the HNH domain converts Cas9-derived proteins into nickases. Each nuclease domain may be modified using well-known methods such as site-directed mutagenesis, PCR-mediated mutagenesis and overall gene synthesis, as well as other methods known in the art.

In the present invention, the DNA encoding the Cas protein may be operably linked to at least one promoter control sequence. In some repetitions, the DNA coding sequence may be operably linked to a promoter control sequence for expression in a eukaryotic cell or animal of interest. The promoter control sequence may be structural, regulated or tissue-specific. Suitable structural promoter control sequences include, but are not limited to, cytomegalovirus early promoters (CMV), simian virus (SV40) promoters, adenovirus major late promoters, Rouse sarcoma virus (RSV) promoters, mouse mammary tumor virus (MMTV) promoters, phosphoglycerate kinase (PGK) promoters, elongation factor (ED1)-alpha promoters, ubiquitin promoters, actin promoters, tubulin promoters, immunoglobulin promoters, fragments thereof, or any combinations thereof. Examples of suitable regulated promoter control sequences include, but are not limited to, those regulated by heat shock, metals, steroids, antibiotics or alcohols. Non-limiting examples of tissue specific promoters include B29 promoters, CD14 promoters, CD43 promoters, CD45 promoters, CD68 promoters, desmin promoters, elastase-1 promoters, endoglin promoters, fibrinectin promoters, Flt-1 promoters, GFAP promoters, GPIIb promoters, ICAM-2 promoters, INF-β promoters, Mb promoters, NphsI promoters, OG-2 promoters, SP-B promoters, SYN1 promoters and WASP promoters. Promoter sequences may be wild-type or modified for more efficient or effective expression. In one exemplary embodiment, the encoding DNA may be operably linked to a CMV promoter for structural expression in mammalian cells.

In the present invention, the sequence encoding the Cas protein may be operably linked to a promoter sequence recognized by a phage RNA polymerase for *in-vitro* mRNA synthesis. In such embodiments, the RNA transcribed *in vitro* can be purified and used by well-known methods. For example, the promoter sequence may be a mutation of the T7, T3 or SP6 promoter sequence or the T7, T3 or SP6 promoter sequence. In an exemplary embodiment, the DNA encoding the Cas protein is operably linked to the T7 promoter for *in-vitro* mRNA synthesis using T7 RNA polymerase.

In alternative embodiments, the sequence encoding the Cas protein may be operably linked to a promoter sequence for *in-vitro* expression of the Cas protein in bacterial or eukaryotic cells. In such embodiments, the expressed protein can be purified and used by known methods. Suitable bacterial promoters include, but are not limited to, T7 promoters, lac operon promoters, trp promoters, variants thereof and combinations thereof. An exemplary bacterial promoter is tac, which is a hybrid of the trp and lac promoters. Non-limiting examples of suitable eukaryotic promoters are listed above. In a further aspect, the DNA encoding the Cas protein may also be linked to a polyadenylation signal (e.g., an SV40 polyA signal or a bovine growth hormone (BGH) polyA signal) and/or at least one transcription termination sequence.

In various embodiments, the DNA encoding Cas protein may be present in the vector. Suitable vectors include plasmid vectors, phagemids, cosmids, artificial/mini chromosomes, transposons and viral vectors (e.g., lentiviral vectors, adeno-associated virus vectors). In an exemplary embodiment, the DNA encoding Cas protein is present in a plasmid vector. Non-limiting examples of suitable plasmid vectors include pUC, pBR322, pET, pBluescript and variants thereof. The vectors may include additional expression control sequences (e.g., enhancer sequences, Kozak sequences, polyadenylation sequences, transcription termination sequences), selectable marker sequences (e.g., antibiotic-resistant genes), origins of replication, and the like. Additional information is provided in "Current Protocols in Molecular Biology" Ausubel et al., John Wiley & Sons, New York, 2003 or "Molecular Cloning: A Laboratory Manual" Sambrook & Russell, Cold Spring Harbor Press, Cold Spring Harbor, NY, 3rd edition, 2001.

The Cas protein, along with the guide RNA, is directed to the target site within the chromosomal sequence, where the Cas protein introduces a double-stranded break in the chromosome sequence. The target site is not limited with regard to sequence, except that the sequence is right followed by a (downstream) consensus sequence. This consensus sequence is also known as a protospacer adjacent motif (PAM). Examples of the PAM include, but are not limited to, NGG, NGGNG and NNAGAAW, where N is defined as any nucleotide and W is defined as A or T. As detailed above, the first region (at the 5' end) of the guide RNA is complementary to the protospacer of the target sequence.

Typically, the first region of the guide RNA is about 19 to 21 nucleotides in length. Thus, in certain aspects, the sequence of the target site within the chromosomal sequence is 5'-N19-21-NGG-3'. PAM is italicized. The target site may be present in a coding region of a gene, an intron of a gene, a control region of a gene, a noncoding region between genes or the like. The gene may be a protein-coding gene or an RNA-coding gene. The gene may be any gene of interest, and may preferably be factor VIII or IX.

In another aspect, the present invention is directed to a transformed rabbit produced using a CRISPR/Cas9 system including a vector inserted with a polynucleotide encoding an sgRNA including a targeting domain that complementarily binds to a part of the exon region of factor VIII (FVIII) or factor IX (FIX).

In another aspect, the transgenic rabbit is produced by a method including:
(a) transcribing the CRISPR/Cas9 system according to the present invention to produce sgRNA and Cas9 mRNA;
(b) introducing the mRNA produced in step (a) into an embryo and culturing the embryo; and
(c) transplanting the embryo obtained in step (b) to a surrogate mother to produce the transgenic rabbit.

In the present invention, the transgenic rabbit may be produced by a method further including determining whether or not transformation occurs after the rabbit production.

In another aspect, the present invention is directed to a transgenic rabbit progeny produced by a method including crossing the transgenic rabbit to produce the transgenic rabbit progeny.

In the present invention, "progeny" refers to any viable transgenic rabbit offspring obtained by crossing with the transgenic rabbit, and more specifically, may be an F1 generation produced by crossing the transgenic rabbit with another transgenic rabbit as parents, an F2 generation obtained by crossing the carrier rabbit of the F1 generation with a normal rabbit, or a subsequent generation, but is not limited thereto.

In the present invention, the crossing may be carried out by crossing with the transgenic rabbit or a normal rabbit.

In the present invention, the transgenic rabbit or transgenic rabbit progeny may exhibit a hemophilia phenotype since factor VIII or factor IX is knocked out therefrom.

The present invention is also directed to cells, tissues and byproducts isolated from the transgenic rabbit or transgenic rabbit progeny.

In the present invention, the byproduct is any substance derived from the transgenic rabbit, but may be preferably selected from the group consisting of blood, serum, urine, feces, saliva, organs and skin, but is not limited thereto.

In another aspect, the present invention is directed to a method of producing a transgenic rabbit from which factor VIII or factor IX is knocked out, including:
(a) transcribing the CRISPR/Cas9 system according to the present invention to produce sgRNA and Cas9 mRNA;
(b) introducing the mRNA produced in step (a) into an embryo and culturing the embryo; and
(c) transplanting the embryo obtained in step (b) to a surrogate mother to produce the transgenic rabbit.

The present invention is also directed to a method for producing a transgenic rabbit progeny including crossing the transgenic rabbit produced by the production method to produce a transgenic rabbit progeny.

In the present invention, the crossing may be carried out by crossing with the transgenic rabbit or a normal rabbit.

The present invention is also directed to a transgenic rabbit that exhibits a hemophilia A phenotype as desired since factor VIII is knocked out therefrom, and a method for producing the same.

The present invention is also directed to a transgenic rabbit that exhibits a hemophilia B phenotype as desired by knocking out factor IX therefrom, and a method for producing the same.

The present invention is also directed to a transgenic rabbit that is produced by crossing a rabbit from which factor VIII is knocked out or a rabbit from which factor IX is knocked out, and is thus used to study the immune response upon injection of the human factor VIII or IX, and a method of producing the same.

The rabbit from which factor VIII or factor IX is knocked out shows no activity related to factor VIII or factor IX thereof, and is thus useful for studying immune responses upon injection of the human factor VIII or IX and the development of hemophilia drugs.

Hereinafter, the present invention will be described in more detail with reference to examples. However, it will be obvious to those skilled in the art that these examples are provided only for illustration of the present invention and should not be construed as limiting the scope of the present invention.

### Example 1. sgRNA design and CRISPR/Cas9 vector construction and in-vitro transcription

### 1-1. sgRNA design

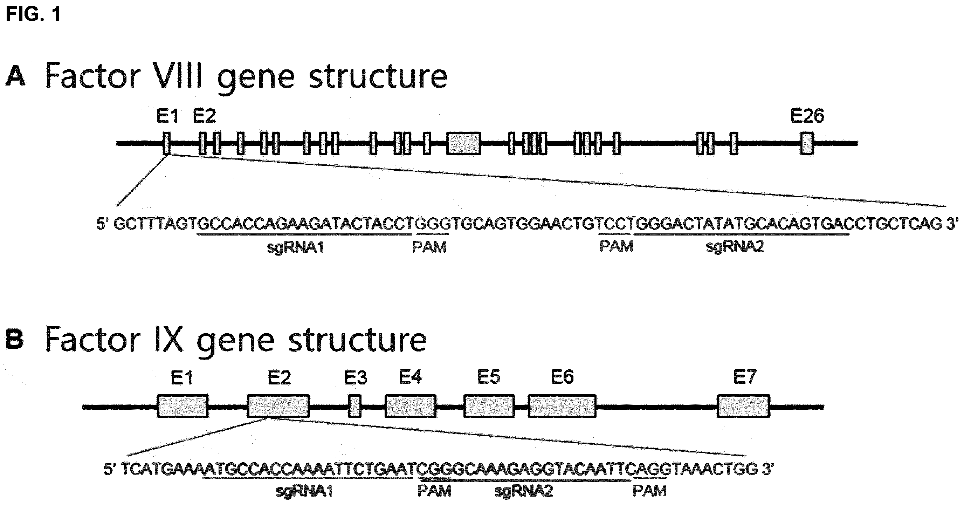

In the case of factor VIII, sgRNAs represented by the nucleotide sequences of SEQ ID NO: 3 and SEQ ID NO: 4 were designed using the sequence represented by the following SEQ ID NO: 1 in the exon 1 region (FIG. 1). 
SEQ ID NO 1:
ATGCAAATAGAGCTCTCCACCTGTTTCTTTGTGTGTATTTTACAATTGAGC
TTTAGTGCCACCAGAAGATACTACCTGGGTGCAGTGAACTGTCCTGGGACTATATGCAC
AGTGAC CTGCTCAGTGA
SEQ ID NO 3: sgRNA 1 (+ Strand)
   5'- GCCACCAGAAGATACTACCTGGG -3'
SEQ ID NO 4: sgRNA 2 (- Strand)
   5'- GTCACTGTGCATATAGTCCCAGG -3'

In the case of factor IX, sgRNAs represented by the nucleotide sequences of SEQ ID NO: 5 and SEQ ID NO: 6 were designed using the sequence represented by the following SEQ ID NO: 2 in the exon 2 region. 
SEQ ID NO 2:

TTTTTCTTGATCATGAAAATGCCACCAAAATTCTGAATCGGGCAAAGAGGT

ACAATTCAGGTAAACTGGAAGAGTTTGTTTCAGGGAACCTTGAGAGAGAATGTATAGAA

GAAAGGTGTAGTTTTGAAGAAGCTCGAGAAGTTTTTGAAAACACTGAAAAAACT
SEQ ID NO 5: sgRNA 1 (+ Strand)
   5'- ATGCCACCAAAATTCTGAATCGG -3'
SEQ ID NO 6: sgRNA 2 (+ Strand)
   5' CGGGCAAAGAGGTACAATTCAGG -3'

### 1-2. sgRNA and Cas9 mRNA transcription

Oligonucleotides suitable for the sgRNA sequences designed in Example 1-1 were designed and cloned into the pUC57-T7 vector (Addgene ID 51306), and the completed sgRNA and the T7 promoter located therein were amplified by PCR with the primers of SEQ ID NOS: 7 and 8.

The PCR amplification product was obtained using T7 RNA polymerase (MAIXscript T7 Kit, Ambion) and then purified (miRNeasy Mini Kit, Qiagen).
SEQ ID NO 7: T7-F
   5'- GAAATTAATACGACTCACTAT -3'
SEQ ID NO 8: T7-R
   5'- AAAAAAAGCACCGACTCGGTGCCAC -3'

In the case of Cas9 mRNA, the Cas9 expression vector was linearized, and then capped mRNA was produced using a mMessage mMachine SP6 Kit (Ambion) and purified using an RNeasy Mini Kit (Qiagen).

### Example 2. Injection of transcribed Cas9/sgRNA into embryos

The Cas9/FVIII sgRNA or Cas9/FIX sgRNA obtained in Example 1 was introduced into fertilized rabbit eggs using a known method (Sci Rep. 2016; 6:222024).

ApproIXmately 18 to 20 hours after fertilization, the fertilized rabbit eggs were transferred to a embryo culture medium (9.5g TCM-119, 0.05g NaHCO₃ (Sigma, S4019), 0.75g Hepes (Sigma H3784), 0.05g penicillin, 0.06g streptomycin, 1.755g NaCl, 3.0g BSA and 1L Milli Q distilled water), FVIII sgRNA (25 ng/µl) and Cas9 mRNA (100 ng/µl) or FIX sgRNA (25 ng/µl) and Cas9 mRNA (100 ng/µl) were injected into the embryo cytoplasm and then the embryo cytoplasm was cultured in a culture medium at 5% carbon dioIXde for 30 to 60 minutes at 38.5°C, and then the embryo was transplanted into a surrogate mother to produce a rabbit.

### Example 3. Genotyping of transgenic rabbits

### 3-1. Genotyping of Factor VIII knockout rabbit

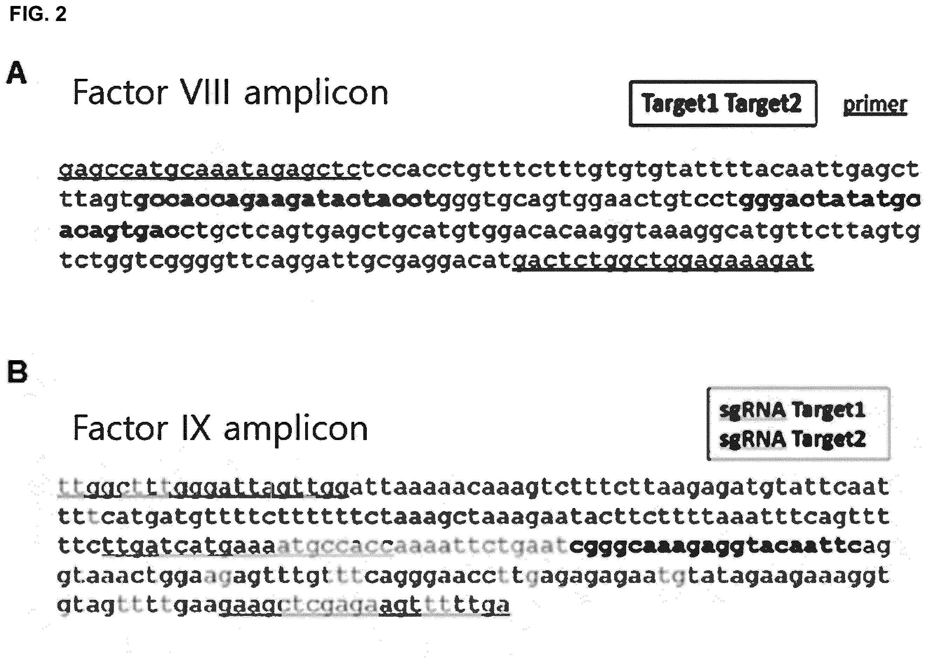

The amplicons shown in (a) of FIG. 2 were amplified using the primers of SEQ ID NOS: 9 and 10, adaptors and tags were attached using secondary and tertiary PCR, deep sequencing was performed using MiSeq (Illumina, MiSeq Reagent Kit V), and the results were analyzed using Cas-Analyzer (Bioinformatics, 2017 Jan 15; 333 (2): 286-288).
SEQ ID NO 9: F8-F
   5'- gagccatgcaaatagagctc -3'
SEQ ID NO 10: F8-R
   5'- atctttctccagccagagtc -3'

The result showed that indels were detected in the FVIII genes of Subjects 2# and 3#, as shown in Table 1 and FIG. 3.

**[Table 1]**

| Rabbit Sample | | Gene | Total Read | Number of insertion containing reads | Number of deletion containing reads | Number of Indel containing reads (%) | Indel pattern |
|---|---|---|---|---|---|---|---|
| **#2** | **Blood** | **F8** | **61151** | **0** | **61128** | **61128 (100.0%)** | **-4** |
| **#3** | **Blood** | **F8** | **55016** | **0** | **54919** | **54919 (99.8%)** | **-3, -12** |

In other words, a mutation in which nucleic acid fragment 4 bp long was deleted was detected in Subject #2, causing premature stop codons and nonsense-mediated decay and thus inhibiting gene expression. Mutations in which nucleic acid fragments 3bp and 12bp long were deleted were detected in Subject #3 (FIG. 3).

### 3-2. Genotyping of factor IX knockout rabbits

The amplicons shown in (b) of FIG. 2 were amplified using the primers of SEQ ID NOS: 11 and 12, adaptors and tags were attached using secondary and tertiary PCR, deep sequencing was performed using MiSeq (Illumina, MiSeq Reagent Kit V), and the results were analyzed using Cas-Analyzer (Bioinformatics, 2017 Jan 15; 333 (2) : 286-288). Subject #5 was primarily amplified using the F9-R2 primer of SEQ ID NO. 13.
SEQ ID NO 11: F9-F
   5'- ttggctttgggattagttgg -3'
SEQ ID NO 12: F9-R
   5'- tcaaaaacttctcgagcttc -3'
SEQ ID NO 13: F9-R2
   5'- tctctgtctgtaactctacc -3'

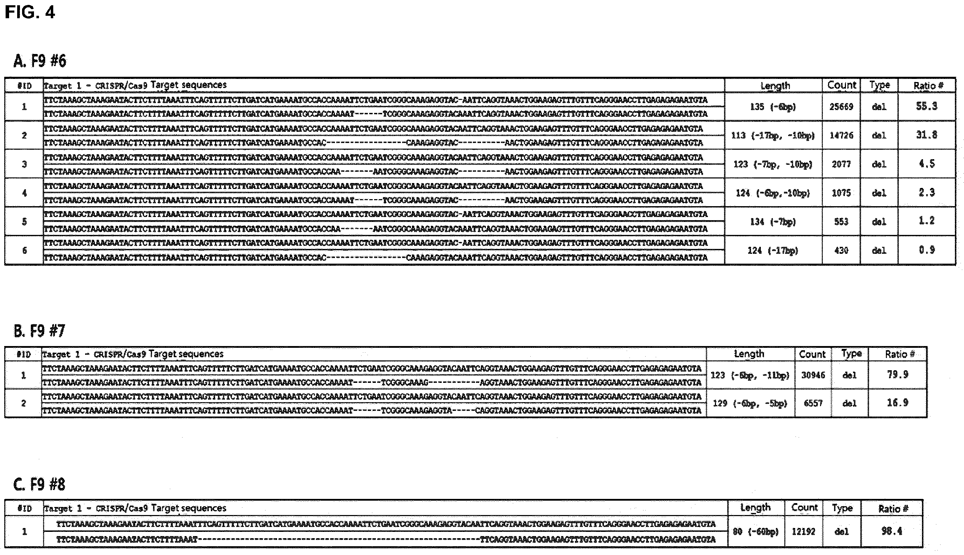

The result showed that Indel was detected from the FIX genes of Subjects #5, #6, #7, #8, #9, #11, #12 and #13, as shown in Table 2 and FIGS. 4 to 6.

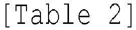

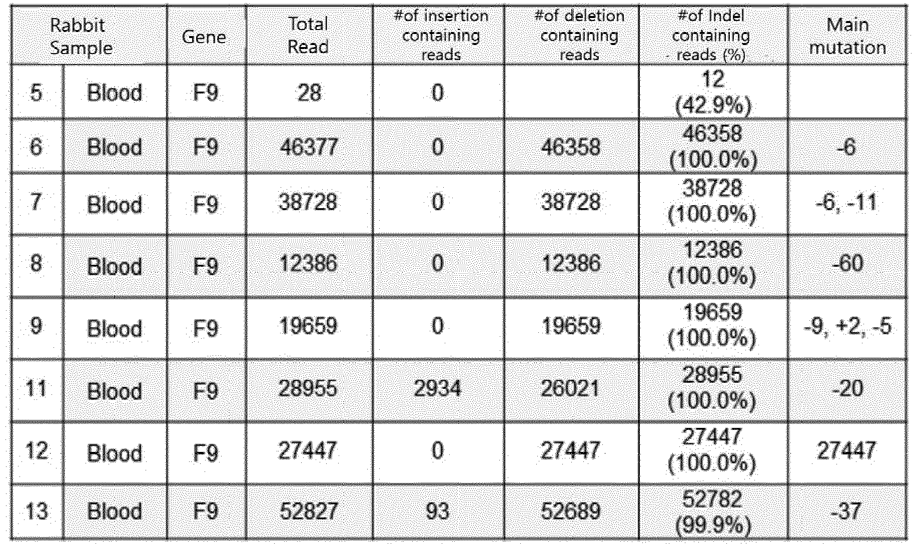

### Example 4. Hemophilia phenotyping of transgenic rabbits

### 4-1. Activated partial thromboplastin time (APTT) analysis

In order to perform the APTT analysis, waveforms of APTT clots were observed in real time using Start 4Hemostasis Analyzer (Stago Inc., USA). That is, 50 µl of APTT reagent (Dade® Actin FSL, Siemens Medical Solutions Inc., USA) solution and 50 µl of plasma isolated from the transgenic rabbit were mixed in a cuvette and incubated at 37°C for 3 minutes, 50 µl of an aqueous calcium chloride (CaCl₂) solution (final concentration: 25 mM) was injected into the cuvette and then the time at which clots were formed was measured.

As a result, as can be seen from FIG. 7A, the clot formation time was 19.4 ± 1.1 seconds for the FVIII knockout rabbit and was 18.9 ± 2.5 seconds for the FIX knockout rabbit. These times indicate that the two rabbits suffer from hemophilia.

### 4-2. Thrombin generation assay (TGA)

Thrombin production was measured by analyzing Fluoroskan Ascent (Thermo Scientific) fluorescent plate readers with Thrombinoscope BV software. That is, 80 µl of a transgenic rabbit plasma and 2 µl of PPP-reagent LOW containing tissue factor and phospholipid were mixed and cultured on a 37°C Immulon microtiter 2HB-high binding 96-well plate (Thermo Nunc) . A mixture of 80 µl of rabbit plasma and 20 µl of thrombin calibrator reagent was cultured in a control well, and a fluorescent thrombin substrate and a preheated Flu-Ca reagent were injected and mixed to homogeneity before the reaction. 20 µl of Flu-Ca reagent was injected to start the reaction and the amount of thrombin produced was analyzed with Thrombinoscope Analysis Version 3.0.

As a result, as shown in B of FIG. 7, 36.9 ± 1.8 nM of thrombin was produced in the control group, while 0.1 ± 0.1 nM of thrombin was produced in the FVIII knockout transgenic rabbit and no thrombin was produced in the FIX knockout transgenic rabbit.

Therefore, the transgenic rabbits from which the FVIII or FIX genes were removed were found to have a hemophilia phenotype.

### Example 5. Breeding pedigree for F1 and F2 generation production

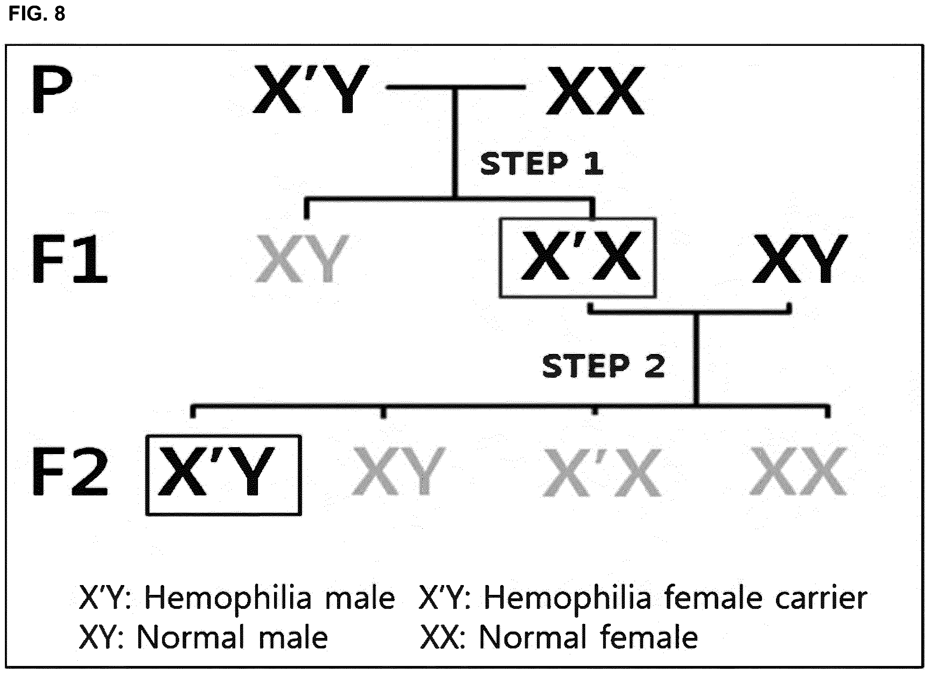

The hemophilia rabbit prepared in Example 2 was called "P" (or F0), and the following process was conducted to obtain F1 and F2 progeny thereof. In order to obtain the progeny of the transgenic rabbit from which factor VIII or IX was knocked out, the subjects were bred as shown in FIG. 8. That is, since hemophilia is a sex-linked heritable disease in which the hemophilia gene is present on the X chromosome, the factor VIII knockout and factor IX knockout transgenic rabbit father (P, X'Y) prepared in Example 2 was crossed with a normal rabbit female (XX) to produce progeny. The female progeny (F1) obtained from the first cross was identified to be a carrier (X'X) through genetic analysis and then the carrier (X'X) was crossed with a normal male (XY) to produce a male transgenic rabbit (F2, X'Y) from which factor VIII and factor IX were knocked out.

### Example 6. Genotyping of F1-generation transgenic rabbit

### 6-1. Genotyping of factor VIII knockout rabbit carrier

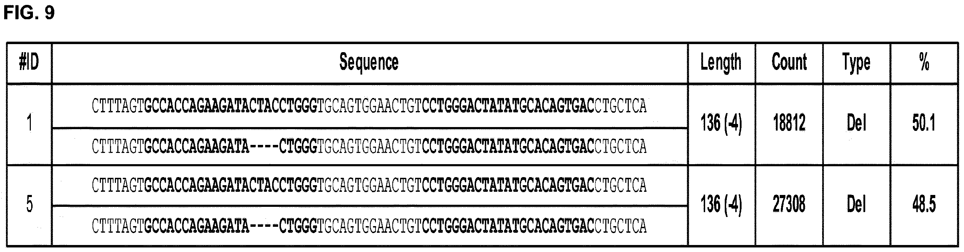

The male rabbit (X'Y), that is, Subject #2, in which the 4bp nucleic acid deletion mutation shown in Table 1 was detected, was crossed with a normal female (XX) with an age of 10 to 12 weeks and a weight of 2 kg or more purchased from Samtako Inc., to obtain progeny. The progeny was identified to be a female carrier (X'X), the F1 generation through gene analysis in the same manner as in Example 3-1. As can be seen from Table 3 and FIG. 9, the indel patterns were detected in the FVIII genes of Subjects #1 and #5.

**[Table 3]**

| Rabbit Sample | | Gene | Total Read | Number of insertion containing reads | Number of deletion containing reads | Number of Indel containing reads (%) | Indel pattern |
|---|---|---|---|---|---|---|---|
| #1 | Blood | F8 | 37549 | 0 | 18812 | 18812(50.1%) | -4 |
| #5 | Blood | F8 | 56305 | 0 | 27308 | 27308(48.5%) | -4 |

### 6-2. Genotyping of factor IX knockout rabbit carrier

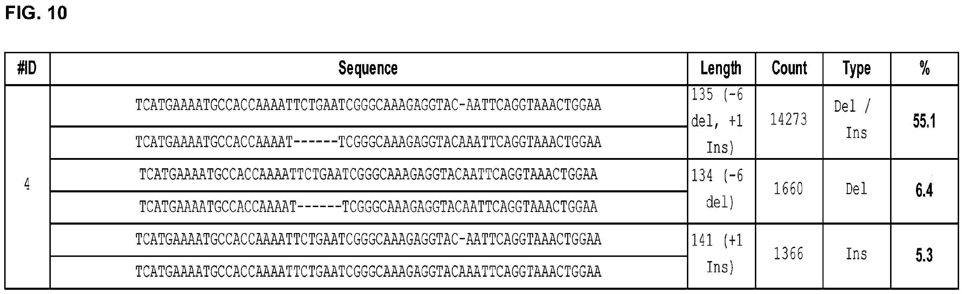

Male (X'Y), that is, Subject #6, having the 6bp nucleic acid deletion mutation shown in Table 2, was crossed with a normal female (XX) to obtain F1 progeny, and the F1 progeny was identified to be a female carrier (X'X) through genetic analysis in the same manner as in Example 3-2. As shown in Table 4 and FIG. 10, indel patterns were detected in the FIX gene of Subject #4, and the indel patterns were a -6bp deletion and a 1bp insertion.

**[Table 4]**

| Rabbit Sample | | Gene | Total Read | Number of insertion containing reads | Number of deletion containing reads | Number of Indel containing reads (%) | Indel pattern |
|---|---|---|---|---|---|---|---|
| #4 | Blood | F9 | 17,299 | 0 | 4,587 | 4,587(66.8%) | -6, +1 |

### Example 7. Genotyping of F2-generation transgenic rabbit

### 7-1. Genotyping of factor VIII knockout rabbit carrier

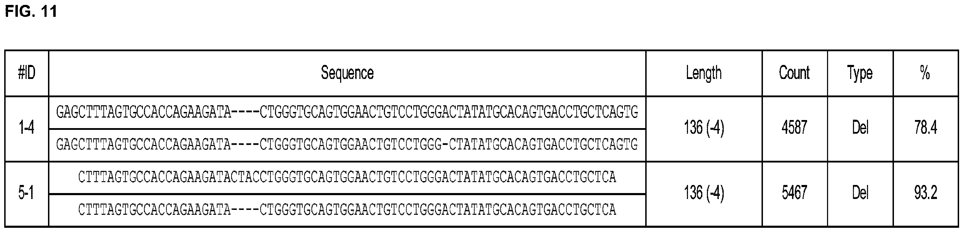

The carrier female (X'X), the F1 generation was crossed with a normal male (XY) with an age of 10 to 12 weeks and a weight of 2 kg or more, purchased from Samtako Inc., to obtain an F2 male (X'Y). The F2 male (X'Y) was identified to be a female carrier (X'X), F1 generation, through gene analysis in the same manner as in Example 3-1. As can be seen from Table 5 and FIG. 11, the indel patterns were detected from the FVIII genes of Subjects #1-4 and #5-1.

**[Table 5]**

| Rabbit Sample | | Gene | Total Read | Number of insertion containing reads | Number of deletion containing reads | Number of Indel containing reads (%) | Indel pattern |
|---|---|---|---|---|---|---|---|
| #1-4 | Blood | F8 | 5,850 | 0 | 4,587 | 4,587(78.4%) | -4 |
| #5-1 | Blood | F8 | 5,865 | 0 | 5,467 | 5,467(93.2%) | -4 |

### 7-2. Genotyping of factor IX knockout rabbit carrier

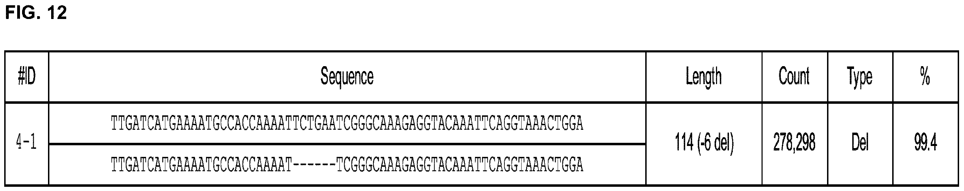

An F2 male (X'Y) obtained by crossing the carrier female (X'X), F1 generation, with a normal male (XY) was subjected to gene analysis in the same manner as in Example 3-2. As can be seen from Table 6 and FIG. 12, indel patterns were detected from the FIX gene of Subject #4-1 and the indel patterns included 6bp, 7bp and 27bp deletions, but the 7bp and 27bp deletions were present in 0.023% and 0.001%, respectively, and thus were considered to be sequencing errors. Thus, Subject #4-1 was identified to be a 6bp nucleic acid deletion mutant.

**[Table 6]**

| Rabbit Sample | | Gene | Total Read | Number of insertion containing reads | Number of deletion containing reads | Number of Indel containing reads (%) | Indel pattern |
|---|---|---|---|---|---|---|---|
| #4-1 | Blood | F9 | 279,860 | 0 | 278,366 | 278,298(99.4%) | -6 |

### Example 8. Phenotyping of F2-generation transgenic rabbit

### 8-1. Claw bleeding model

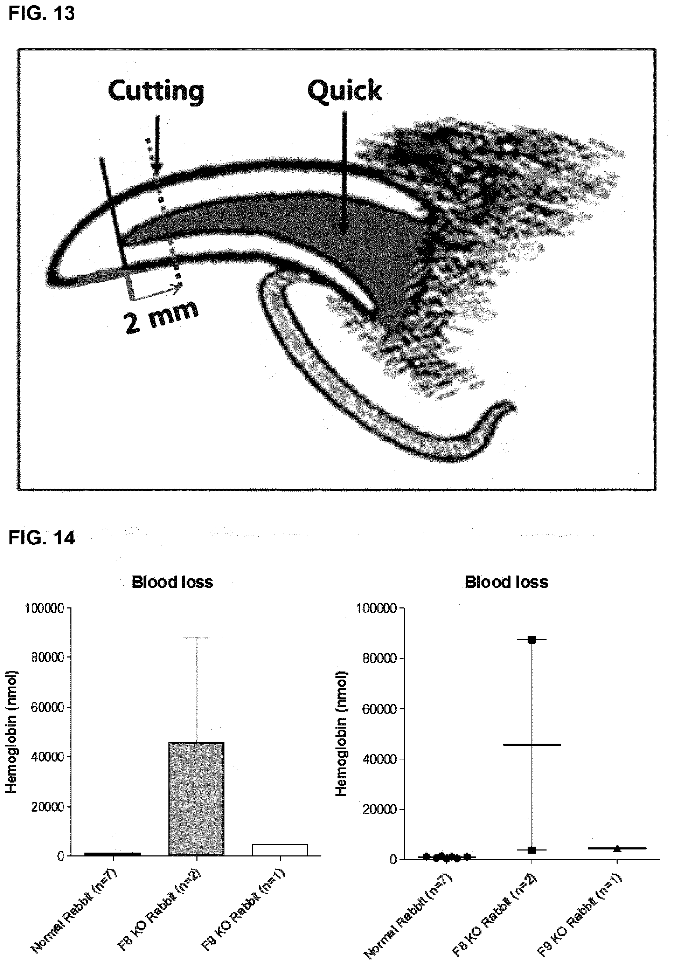

In order to induce a claw bleeding model in rabbits, hemophilia rabbits of Example 7 with an age of 12 weeks or more and a weight of 2 kg or more and normal rabbits with an age of 10 to 12 weeks and a weight of 2 kg or more purchased from Samtako Inc. were anesthetized by injection of 0.4 mg/kg of diazepam and 25 mg/kg of pentobarbital sodium into the auricular vein thereof. One anterior paw of the anesthetized rabbit was depilated with a hair clipper, and a 2 mm proIXmal portion from the quick distal end of the middle toe claw was marked using an oil pen and a Digimatic caliper and then cut using a wire cutter (FIG. 13). Immediately after cutting the claw, the claw was put into a 50 mL conical tube containing a sterile saline solution maintained at 37°C for 60 minutes, the blood was collected for 60 minutes, the supernatant was removed by centrifugation at 1500 x g for 5 minutes, and tertiary distilled water was added up to 20 mL to a conical tube using a 10 mL pipette, and the blood was completely hemolyzed in a vortex.

### 8-2. Hemoglobin assay (HB) analysis

The amount of hemoglobin in the blood was quantified with a hemoglobin assay kit (Sigma and Aldrich, MAK115-1KT, # BF03A26V) using the sample hemolyzed in Example 8-1 to measure blood loss. It was found that the concentration of hemoglobin of normal rabbits was 1,014 nM (range: 502-1503), the concentration of hemoglobin of the factor VIII knockout rabbit was 45,787 nM, and the hemoglobin concentration of the factor IX knockout rabbit was 4,620 nM (FIG. 14).

Although specific configurations of the present invention have been described in detail, those skilled in the art will appreciate that this description is provided to set forth preferred embodiments for illustrative purposes and should not be construed as limiting the scope of the present invention. Therefore, the substantial scope of the present invention is defined by the accompanying claims and equivalents thereto.

### [Industrial Applicability]

The transgenic rabbit, from which factor VIII and/or factor IX is knocked out according to the present invention, is inhibited in the function of factor VIII and/or factor IX, which is a protein that plays an important role in the development of hemophilia, thus being useful for the development of hemophilia drugs or hemophilia research.

### [Sequence Listing Free Text]

An electronic file is attached.

## Claims

1. SgRNA comprising a targeting domain complementarily binding to an exon region of factor VIII (FVIII) or factor IX (FIX).

2. The sgRNA according to claim 1, wherein the exon region of the factor VIII (FVIII) gene is an exon region represented by a base sequence of SEQ ID NO: 1.

3. The sgRNA according to claim 1, wherein the exon region of the factor IX (FIX) gene is an exon region represented by a base sequence of SEQ ID NO: 2.

4. A polynucleotide encoding the sgRNA according to any one of claims 1 to 3.

5. The polynucleotide according to claim 4, wherein the polynucleotide is represented by a base sequence of any one of SEQ ID NOS: 3 to 6.

6. A vector having the polynucleotide according to claim 4 inserted therein.

7. A CRISPR/Cas system comprising the vector according to claim 6.

8. A transgenic rabbit produced using the CRISPR/Cas9 system according to claim 7.

9. The transgenic rabbit according to claim 8, wherein the transgenic rabbit is produced by a method comprising:
(a) transcribing the CRISPR/Cas9 system according to claim 7 to produce sgRNA and Cas9 mRNA;
(b) introducing the mRNA produced in step (a) into an embryo and culturing the embryo; and
(c) transplanting the embryo obtained in step (b) into a surrogate mother to produce the transgenic rabbit.

10. The transgenic rabbit according to claim 9, wherein the transgenic rabbit is produced by a method further comprising determining whether or not transformation occurs after the rabbit production.

11. A transgenic rabbit progeny produced by a method comprising crossing the transgenic rabbit according to claim 9 to produce the transgenic rabbit progeny.

12. The transgenic rabbit progeny according to claim 11, wherein the crossing the transgenic rabbit is carried out by crossing with the transgenic rabbit according to claim 9 or with a normal rabbit.

13. The transgenic rabbit progeny according to any one of claims 8 to 12, wherein the transgenic rabbit or transgenic rabbit progeny exhibits a hemophilia phenotype since factor VIII or factor IX is knocked out.

14. A cell, tissue and byproduct isolated from the transgenic rabbit according to claim 8 or the transgenic rabbit progeny according to claim 11.

15. The cell, tissue and byproduct according to claim 14, wherein the byproduct is selected from the group consisting of blood, serum, urine, feces, saliva, organs and skin.

16. A method of producing a transgenic rabbit comprising:
(a) transcribing the CRISPR/Cas9 system according to claim 7 to produce sgRNA and Cas9 mRNA;
(b) introducing the mRNA produced in step (a) into an embryo and culturing the embryo; and
(c) transplanting the embryo obtained in step (b) into a surrogate mother to produce the transgenic rabbit.

17. A method of producing a transgenic rabbit progeny comprising crossing the transgenic rabbit produced by the method according to claim 16 to produce the transgenic rabbit progeny.

18. The method according to claim 17, wherein the crossing the transgenic rabbit is carried out by crossing with the transgenic rabbit produced by the method according to claim 16 or with a normal rabbit.


<210>    1
<211>    127
<212>    DNA
<213>    Artificial Sequence

<220>
<223>    F8 exon 1


<400>    1
atgcaaatag agctctccac ctgtttcttt gtgtgtattt tacaattgag ctttagtgcc        60

accagaagat actacctggg tgcagtgaac tgtcctggga ctatatgcac agtgacctgc       120

tcagtga                                                                 127


<210>    2
<211>    164
<212>    DNA
<213>    Artificial Sequence

<220>
<223>    F9 exon 2


<400>    2
tttttcttga tcatgaaaat gccaccaaaa ttctgaatcg ggcaaagagg tacaattcag        60

gtaaactgga agagtttgtt tcagggaacc ttgagagaga atgtatagaa gaaaggtgta       120

gttttgaaga agctcgagaa gtttttgaaa acactgaaaa aact                        164


<210>    3
<211>    23
<212>    DNA
<213>    Artificial Sequence

<220>
<223>    F8 sgRNA 1


<400>    3
gccaccagaa gatactacct ggg                                                23


<210>    4
<211>    23
<212>    DNA
<213>    Artificial Sequence

<220>
<223>    F8 sgRNA 2


<400>    4
gtcactgtgc atatagtccc agg                                              23


<210>    5
<211>    23
<212>    DNA
<213>    Artificial Sequence

<220>
<223>    F9 sgRNA 1


<400>    5
atgccaccaa aattctgaat cgg                                              23


<210>    6
<211>    23
<212>    DNA
<213>    Artificial Sequence

<220>
<223>    F9 sgRNA 2


<400>    6
cgggcaaaga ggtacaattc agg                                              23


<210>    7
<211>    21
<212>    DNA
<213>    Artificial Sequence

<220>
<223>    T7-F


<400>    7
gaaattaata cgactcacta t                                                21


<210>    8
<211>    25
<212>    DNA
<213>    Artificial Sequence

<220>
<223>    T7-R


<400>    8
aaaaaaagca ccgactcggt gccac                                            25


<210>    9
<211>    20

<212>    DNA
<213>    Artificial Sequence

<220>
<223>    F8-F


<400>    9
gagccatgca aatagagctc                                                    20


<210>    10
<211>    20
<212>    DNA
<213>    Artificial Sequence

<220>
<223>    F8-R


<400>    10
atctttctcc agccagagtc                                                    20


<210>    11
<211>    20
<212>    DNA
<213>    Artificial Sequence

<220>
<223>    F9-F


<400>    11
ttggctttgg gattagttgg                                                    20


<210>    12
<211>    20
<212>    DNA
<213>    Artificial Sequence

<220>
<223>    F9-R


<400>    12
tcaaaaactt ctcgagcttc                                                    20


<210>    13
<211>    20
<212>    DNA
<213>    Artificial Sequence

<220>
<223>    F9-R2


<400>    13
tctctgtctg taactctacc                                                    20

<210>    14
<211>    76
<212>    DNA
<213>    Artificial Sequence

<220>
<223>    F1_A


<400>    14
gctttagtgc caccagaaga tactacctgg gtgcagtgga actgtcctgg gactatatgc    60

acagtgacct gctcag                                                    76


<210>    15
<211>    59
<212>    DNA
<213>    Artificial Sequence

<220>
<223>    F1_B


<400>    15
tcatgaaaat gccaccaaaa ttctgaatcg ggcaaagagg tacaattcag gtaaactgg     59


<210>    16
<211>    222
<212>    DNA
<213>    Artificial Sequence

<220>
<223>    F2_A


<400>    16
gagccatgca aatagagctc tccacctgtt tctttgtgtg tattttacaa tttgagcttt    60

agtgccacca gaagatacta cctgggtgca gtggaactgt cctgggacta tatgcacagt   120

gacctgctca gtgagctgca tgtggacaca aggtaaaggc atgttcttag tgtctggtcg   180

gggttcagga ttgcgaggac atgactctgg ctggagaaag at                      222


<210>    17
<211>    259
<212>    DNA
<213>    Artificial Sequence

<220>
<223>    F2_B


<400>    17
ttggctttgg gattagttgg attaaaaaca aagtctttct taagagatgt attcaatttt    60

catgatgttt tcttttttct aaagctaaag aatacttctt ttaaatttca gtttttcttg   120

atcatgaaaa tgccaccaaa attctgaatc gggcaaagag gtacaattca ggtaaactgg   180

aagagtttgt ttcagggaac cttgagagag aatgtataga agaaaggtgt agttttgaag 240

aagctcgaga agtttttga 259

<210> 18
<211> 140
<212> DNA
<213> Artificial Sequence

<220>
<223> F3_A_1

<400> 18
aaatagagct ctccacctgt ttctttgtgt gtattttaca attgagcttt agtgccacca 60

gaagatacta cctgggtgca gtggaactgt cctgggacta tatgcacagt gacctgctca 120

gtgagctgca tgtggacaca 140

<210> 19
<211> 136
<212> DNA
<213> Artificial Sequence

<220>
<223> F3_A_1-2

<400> 19
aaatagagct ctccacctgt ttctttgtgt gtattttaca attgagcttt agtgccacca 60

gaagatactg ggtgcagtgg aactgtcctg ggactatatg cacagtgacc tgctcagtga 120

gctgcatgtg gacaca 136

<210> 20
<211> 140
<212> DNA
<213> Artificial Sequence

<220>
<223> F3_B_1-1

<400> 20
aaatagagct ctccacctgt ttctttgtgt gtattttaca attgagcttt agtgccacca 60

gaagatacta cctgggtgca gtggaactgt cctgggacta tatgcacagt gacctgctca 120

gtgagctgca tgtggacaca 140

<210> 21
<211> 137
<212> DNA
<213> Artificial Sequence

<220>
<223> F3_B_1-2

<400>    21
aaatagagct ctccacctgt ttctttgtgt gtattttaca attgagcttt agtgccacca    60

gaagatacct gggtgcagtg gaactgtcct gggactatat gcacagtgac ctgctcagtg    120

agctgcatgt ggacaca                                                   137


<210>    22
<211>    140
<212>    DNA
<213>    Artificial Sequence

<220>
<223>    F3_B_2-1


<400>    22
aaatagagct ctccacctgt ttctttgtgt gtattttaca attgagcttt agtgccacca    60

gaagatacta cctgggtgca gtggaactgt cctgggacta tatgcacagt gacctgctca    120

gtgagctgca tgtggacaca                                                140


<210>    23
<211>    128
<212>    DNA
<213>    Artificial Sequence

<220>
<223>    F3_B_2-2


<400>    23
aaatagagct ctccacctgt ttctttgtgt gtattttaca attgagcttt agtgccaccc    60

tgggtgcagt ggaactgtcc tgggactata tgcacagtga cctgctcagt gagctgcatg    120

tggacaca                                                             128


<210>    24
<211>    140
<212>    DNA
<213>    Artificial Sequence

<220>
<223>    F3_B_3-1


<400>    24
aaatagagct ctccacctgt ttctttgtgt gtattttaca attgagcttt agtgccacca    60

gaagatacta cctgggtgca gtggaactgt cctgggacta tatgcacagt gacctgctca    120

gtgagctgca tgtggacaca                                                140


<210>    25
<211>    137

<212>    DNA
<213>    Artificial Sequence

<220>
<223>    F3_B_3-2


<400>    25
aaatagagct ctccacctgt ttctttgtgt gtattttaca attgagcttt agtgccacca        60

gaagatacct gggtgcagtg gaactgtcct gggactatat gcacagtgac ctgctcagtg       120

agctgcatgt ggacaca                                                      137


<210>    26
<211>    140
<212>    DNA
<213>    Artificial Sequence

<220>
<223>    F3_B_4-1


<400>    26
aaatagagct ctccacctgt ttctttgtgt gtattttaca attgagcttt agtgccacca        60

gaagatacta cctgggtgca gtggaactgt cctgggacta tatgcacagt gacctgctca       120

gtgagctgca tgtggacaca                                                   140


<210>    27
<211>    138
<212>    DNA
<213>    Artificial Sequence

<220>
<223>    F3_B_4-2


<400>    27
aaatagagct ctccacctgt ttctttgtgt gtattttaca attgagcttt agtgccacca        60

gaagatacct gggtgcagtg gaactgtcct gggaactata tgcacagtga cctgctcagt       120

gagctgcatg tggacaca                                                     138


<210>    28
<211>    140
<212>    DNA
<213>    Artificial Sequence

<220>
<223>    F3_B_5-1


<400>    28
aaatagagct ctccacctgt ttctttgtgt gtattttaca attgagcttt agtgccacca        60

gaagatacta cctgggtgca gtggaactgt cctgggacta tatgcacagt gacctgctca       120

gtgagctgca tgtggacaca                                                140

<210>    29
<211>    136
<212>    DNA
<213>    Artificial Sequence

<220>
<223>    F3_B_5-2

<400>    29
aaatagagct ctccacctgt ttctttgtgt gtattttaca attgagcttt agtgccacca      60

gaagatacct gggtgcagtg gaactgtcct gggctatatg cacagtgacc tgctcagtga     120

gctgcatgtg gacaca                                                    136

<210>    30
<211>    140
<212>    DNA
<213>    Artificial Sequence

<220>
<223>    F4_A_1-1

<400>    30
ttctaaagct aaagaatact tcttttaaat ttcagttttt cttgatcatg aaaatgccac      60

caaaattctg aatcgggcaa agaggtacaa ttcaggtaaa ctggaagagt ttgtttcagg     120

gaaccttgag agagaatgta                                                140

<210>    31
<211>    135
<212>    DNA
<213>    Artificial Sequence

<220>
<223>    F4_A_1-2

<400>    31
ttctaaagct aaagaatact tcttttaaat ttcagttttt cttgatcatg aaaatgccac      60

caaaattcgg gcaaagaggt acaaattcag gtaaactgga agagtttgtt tcagggaacc     120

ttgagagaga atgta                                                     135

<210>    32
<211>    140
<212>    DNA
<213>    Artificial Sequence

<220>
<223>    F4_A_2-1

<400>    32
ttctaaagct aaagaatact tcttttaaat ttcagttttt cttgatcatg aaaatgccac    60

caaaattctg aatcgggcaa agaggtacaa ttcaggtaaa ctggaagagt ttgtttcagg    120

gaaccttgag agagaatgta                                                140


<210>    33
<211>    113
<212>    DNA
<213>    Artificial Sequence

<220>
<223>    F4_A_2-2


<400>    33
ttctaaagct aaagaatact tcttttaaat ttcagttttt cttgatcatg aaaatgccac    60

caaagaggta caactggaag agtttgtttc agggaacctt gagagagaat gta           113


<210>    34
<211>    140
<212>    DNA
<213>    Artificial Sequence

<220>
<223>    F4_A_3-1


<400>    34
ttctaaagct aaagaatact tcttttaaat ttcagttttt cttgatcatg aaaatgccac    60

caaaattctg aatcgggcaa agaggtacaa ttcaggtaaa ctggaagagt ttgtttcagg    120

gaaccttgag agagaatgta                                                140


<210>    35
<211>    123
<212>    DNA
<213>    Artificial Sequence

<220>
<223>    F4_A_3-2


<400>    35
ttctaaagct aaagaatact tcttttaaat ttcagttttt cttgatcatg aaaatgccac    60

caaaatcggg caaagaggta caactggaag agtttgtttc agggaacctt gagagagaat    120

gta                                                                  123


<210>    36
<211>    140
<212>    DNA
<213>    Artificial Sequence

<220>

<223>    F4_A_4-1

<400>    36
ttctaaagct aaagaatact tcttttaaat ttcagttttt cttgatcatg aaaatgccac    60

caaaattctg aatcgggcaa agaggtacaa ttcaggtaaa ctggaagagt ttgtttcagg    120

gaaccttgag agagaatgta    140

<210>    37
<211>    124
<212>    DNA
<213>    Artificial Sequence

<220>
<223>    F4_A_4-2

<400>    37
ttctaaagct aaagaatact tcttttaaat ttcagttttt cttgatcatg aaaatgccac    60

caaaattcgg gcaaagaggt acaactggaa gagtttgttt cagggaacct tgagagagaa    120

tgta    124

<210>    38
<211>    140
<212>    DNA
<213>    Artificial Sequence

<220>
<223>    F4_A_5-1

<400>    38
ttctaaagct aaagaatact tcttttaaat ttcagttttt cttgatcatg aaaatgccac    60

caaaattctg aatcgggcaa agaggtacaa ttcaggtaaa ctggaagagt ttgtttcagg    120

gaaccttgag agagaatgta    140

<210>    39
<211>    134
<212>    DNA
<213>    Artificial Sequence

<220>
<223>    F4_A_5-2

<400>    39
ttctaaagct aaagaatact tcttttaaat ttcagttttt cttgatcatg aaaatgccac    60

caaaatcggg caaagaggta caaattcagg taaactggaa gagtttgttt cagggaacct    120

tgagagagaa tgta    134

<210>    40

<211>    140
<212>    DNA
<213>    Artificial Sequence

<220>
<223>    F4_A_6-1


<400>    40
ttctaaagct aaagaatact tcttttaaat ttcagttttt cttgatcatg aaaatgccac        60

caaaattctg aatcgggcaa agaggtacaa ttcaggtaaa ctggaagagt ttgtttcagg       120

gaaccttgag agagaatgta                                                   140


<210>    41
<211>    124
<212>    DNA
<213>    Artificial Sequence

<220>
<223>    F4_A_6-2


<400>    41
ttctaaagct aaagaatact tcttttaaat ttcagttttt cttgatcatg aaaatgccac        60

caaagaggta caaattcagg taaactggaa gagtttgttt cagggaacct tgagagagaa       120

tgta                                                                    124


<210>    42
<211>    140
<212>    DNA
<213>    Artificial Sequence

<220>
<223>    F4_B_1-1


<400>    42
ttctaaagct aaagaatact tcttttaaat ttcagttttt cttgatcatg aaaatgccac        60

caaaattctg aatcgggcaa agaggtacaa ttcaggtaaa ctggaagagt ttgtttcagg       120

gaaccttgag agagaatgta                                                   140


<210>    43
<211>    123
<212>    DNA
<213>    Artificial Sequence

<220>
<223>    F4_B_1-2


<400>    43
ttctaaagct aaagaatact tcttttaaat ttcagttttt cttgatcatg aaaatgccac        60

caaaattcgg gcaaagaggt aaactggaag agtttgtttc agggaacctt gagagagaat       120

gta                                                                 123

<210>    44
<211>    140
<212>    DNA
<213>    Artificial Sequence

<220>
<223>    F4_B_2-1

<400>    44
ttctaaagct aaagaatact tcttttaaat ttcagttttt cttgatcatg aaaatgccac        60

caaaattctg aatcgggcaa agaggtacaa ttcaggtaaa ctggaagagt ttgtttcagg       120

gaaccttgag agagaatgta                                                   140

<210>    45
<211>    129
<212>    DNA
<213>    Artificial Sequence

<220>
<223>    F4_B_2-2

<400>    45
ttctaaagct aaagaatact tcttttaaat ttcagttttt cttgatcatg aaaatgccac        60

caaaattcgg gcaaagaggt acaggtaaac tggaagagtt tgtttcaggg aaccttgaga       120

gagaatgta                                                               129

<210>    46
<211>    140
<212>    DNA
<213>    Artificial Sequence

<220>
<223>    F4_C_1-1

<400>    46
ttctaaagct aaagaatact tcttttaaat ttcagttttt cttgatcatg aaaatgccac        60

caaaattctg aatcgggcaa agaggtacaa ttcaggtaaa ctggaagagt ttgtttcagg       120

gaaccttgag agagaatgta                                                   140

<210>    47
<211>    80
<212>    DNA
<213>    Artificial Sequence

<220>
<223>    F4_C_1-2

<400>    47
ttctaaagct aaagaatact tcttttaaat ttcaggtaaa ctggaagagt ttgtttcagg        60

gaaccttgag agagaatgta                                                    80


<210>    48
<211>    140
<212>    DNA
<213>    Artificial Sequence

<220>
<223>    F5_A_1-1


<400>    48
ttctaaagct aaagaatact tcttttaaat ttcagttttt cttgatcatg aaaatgccac        60

caaaattctg aatcgggcaa agaggtacaa ttcaggtaaa ctggaagagt ttgtttcagg       120

gaaccttgag agagaatgta                                                   140


<210>    49
<211>    128
<212>    DNA
<213>    Artificial Sequence

<220>
<223>    F5_A_1-2


<400>    49
ttctaaagct aaagaatact tcttttaaat ttcagttttt cttgatcatg aaaatgccac        60

caagaggtgg caaagaggta caggtaaact ggaagagttt gtttcaggga accttgagag       120

agaatgta                                                                128


<210>    50
<211>    140
<212>    DNA
<213>    Artificial Sequence

<220>
<223>    F5_A_2-1


<400>    50
ttctaaagct aaagaatact tcttttaaat ttcagttttt cttgatcatg aaaatgccac        60

caaaattctg aatcgggcaa agaggtacaa ttcaggtaaa ctggaagagt ttgtttcagg       120

gaaccttgag agagaatgta                                                   140


<210>    51
<211>    114
<212>    DNA
<213>    Artificial Sequence

<220>
<223>    F5_A_2-2


<400>    51
ttctaaagct aaagaatact tcttttaaat ttcagttttt cttgatcatg aaaatgcaaa         60

gaggtacagg taaactggaa gagtttgttt cagggaacct tgagagagaa tgta              114


<210>    52
<211>    140
<212>    DNA
<213>    Artificial Sequence

<220>
<223>    F5_A_3-1


<400>    52
ttctaaagct aaagaatact tcttttaaat ttcagttttt cttgatcatg aaaatgccac         60

caaaattctg aatcgggcaa agaggtacaa ttcaggtaaa ctggaagagt ttgtttcagg        120

gaaccttgag agagaatgta                                                    140


<210>    53
<211>    128
<212>    DNA
<213>    Artificial Sequence

<220>
<223>    F5_A_3-2


<400>    53
ttctaaagct aaagaatact tcttttaaat ttcagttttt cttgatcatg aaaatgccac         60

caaaatcggg caaagaggta caggtaaact ggaagagttt gtttcaggga accttgagag        120

agaatgta                                                                 128


<210>    54
<211>    140
<212>    DNA
<213>    Artificial Sequence

<220>
<223>    F5_A_4-1


<400>    54
ttctaaagct aaagaatact tcttttaaat ttcagttttt cttgatcatg aaaatgccac         60

caaaattctg aatcgggcaa agaggtacaa ttcaggtaaa ctggaagagt ttgtttcagg        120

gaaccttgag agagaatgta                                                    140


<210>    55
<211>    125

<212>    DNA
<213>    Artificial Sequence

<220>
<223>    F5_A_4-2


<400>    55
ttctaaagct aaagaatact tcttttaaat ttcagttttt cttgatcatg aaaatgccac        60

caaaaaatcg ggcaaagagg tacaactgga agagtttgtt tcagggaacc ttgagagaga       120

atgta                                                                   125


<210>    56
<211>    140
<212>    DNA
<213>    Artificial Sequence

<220>
<223>    F5_A_5-1


<400>    56
ttctaaagct aaagaatact tcttttaaat ttcagttttt cttgatcatg aaaatgccac        60

caaaattctg aatcgggcaa agaggtacaa ttcaggtaaa ctggaagagt ttgtttcagg       120

gaaccttgag agagaatgta                                                   140


<210>    57
<211>    124
<212>    DNA
<213>    Artificial Sequence

<220>
<223>    F5_A_5-2


<400>    57
ttctaaagct aaagaatact tcttttaaat ttcagttttt cttgatcatg aaaatgccac        60

caaaaaatcg ggcaaagagg taaactggaa gagtttgttt cagggaacct tgagagagaa       120

tgta                                                                    124


<210>    58
<211>    140
<212>    DNA
<213>    Artificial Sequence

<220>
<223>    F5_A_6-1


<400>    58
ttctaaagct aaagaatact tcttttaaat ttcagttttt cttgatcatg aaaatgccac        60

caaaattctg aatcgggcaa agaggtacaa ttcaggtaaa ctggaagagt ttgtttcagg       120

gaaccttgag agagaatgta                                                140

<210>    59
<211>    130
<212>    DNA
<213>    Artificial Sequence

<220>
<223>    F5_A_6-2

<400>    59
ttctaaagct aaagaatact tcttttaaat ttcagttttt cttgatcatg aaaatgccac        60

caaaaaatcg ggcaaagagg tacaggtaaa ctggaagagt ttgtttcagg gaaccttgag       120

agagaatgta                                                          130

<210>    60
<211>    140
<212>    DNA
<213>    Artificial Sequence

<220>
<223>    F5_A_7-1

<400>    60
ttctaaagct aaagaatact tcttttaaat ttcagttttt cttgatcatg aaaatgccac        60

caaaattctg aatcgggcaa agaggtacaa ttcaggtaaa ctggaagagt ttgtttcagg       120

gaaccttgag agagaatgta                                                140

<210>    61
<211>    129
<212>    DNA
<213>    Artificial Sequence

<220>
<223>    F5_A_7-2

<400>    61
ttctaaagct aaagaatact tcttttaaat ttcagttttt cttgatcatg aaaatgccac        60

caaaattcgg gcaaagaggt acaggtaaac tggaagagtt tgtttcaggg aaccttgaga       120

gagaatgta                                                           129

<210>    62
<211>    140
<212>    DNA
<213>    Artificial Sequence

<220>
<223>    F5_A_8-1

<400>    62
ttctaaagct aaagaatact tcttttaaat ttcagttttt cttgatcatg aaaatgccac        60

caaaattctg aatcgggcaa agaggtacaa ttcaggtaaa ctggaagagt ttgtttcagg       120

gaaccttgag agagaatgta                                                   140


<210>    63
<211>    123
<212>    DNA
<213>    Artificial Sequence

<220>
<223>    F5_A_8-2


<400>    63
ttctaaagct aaagaatact tcttttaaat ttcagttttt cttgatcatg aaaatgccac        60

caaaatcggg caaagaggta caactggaag agtttgtttc agggaacctt gagagagaat       120

gta                                                                     123


<210>    64
<211>    140
<212>    DNA
<213>    Artificial Sequence

<220>
<223>    F5_A_9-1


<400>    64
ttctaaagct aaagaatact tcttttaaat ttcagttttt cttgatcatg aaaatgccac        60

caaaattctg aatcgggcaa agaggtacaa ttcaggtaaa ctggaagagt ttgtttcagg       120

gaaccttgag agagaatgta                                                   140


<210>    65
<211>    122
<212>    DNA
<213>    Artificial Sequence

<220>
<223>    F5_A_9-2


<400>    65
ttctaaagct aaagaatact tcttttaaat ttcagttttt cttgatcatg aaaatgccac        60

caaaatcggg caaagaggta aactggaaga gtttgtttca gggaaccttg agagagaatg       120

ta                                                                      122


<210>    66
<211>    140
<212>    DNA
<213>    Artificial Sequence

<220>
<223>    F5_A_10-1


<400>    66
ttctaaagct aaagaatact tcttttaaat ttcagttttt cttgatcatg aaaatgccac         60

caaaattctg aatcgggcaa agaggtacaa ttcaggtaaa ctggaagagt ttgtttcagg        120

gaaccttgag agagaatgta                                                    140


<210>    67
<211>    123
<212>    DNA
<213>    Artificial Sequence

<220>
<223>    F5_A_10-2


<400>    67
ttctaaagct aaagaatact tcttttaaat ttcagttttt cttgatcatg aaaatgccac         60

caagaggtgg caaagaggta caactggaag agtttgtttc agggaacctt gagagagaat        120

gta                                                                      123


<210>    68
<211>    140
<212>    DNA
<213>    Artificial Sequence

<220>
<223>    F5_A_11-1


<400>    68
ttctaaagct aaagaatact tcttttaaat ttcagttttt cttgatcatg aaaatgccac         60

caaaattctg aatcgggcaa agaggtacaa ttcaggtaaa ctggaagagt ttgtttcagg        120

gaaccttgag agagaatgta                                                    140


<210>    69
<211>    109
<212>    DNA
<213>    Artificial Sequence

<220>
<223>    F5_A_11-2


<400>    69
ttctaaagct aaagaatact tcttttaaat ttcagttttt cttgatcatg aaaatgcaaa         60

gaggtacaac tggaagagtt tgtttcaggg aaccttgaga gagaatgta                    109


<210>    70

<211>    140
<212>    DNA
<213>    Artificial Sequence

<220>
<223>    F5_B_1-1


<400>    70
ttctaaagct aaagaatact tcttttaaat ttcagttttt cttgatcatg aaaatgccac        60

caaaattctg aatcgggcaa agaggtacaa ttcaggtaaa ctggaagagt ttgtttcagg       120

gaaccttgag agagaatgta                                                   140


<210>    71
<211>    120
<212>    DNA
<213>    Artificial Sequence

<220>
<223>    F5_B_1-2


<400>    71
ttctaaagct aaagaatact tcttttaaat ttcagttttt cttgatcatg aaaatgccac        60

caaaattctg ttcaggtaaa ctggaagagt ttgtttcagg gaaccttgag agagaatgta       120

                                                                        120


<210>    72
<211>    140
<212>    DNA
<213>    Artificial Sequence

<220>
<223>    F5_B_2-1


<400>    72
ttctaaagct aaagaatact tcttttaaat ttcagttttt cttgatcatg aaaatgccac        60

caaaattctg aatcgggcaa agaggtacaa ttcaggtaaa ctggaagagt ttgtttcagg       120

gaaccttgag agagaatgta                                                   140


<210>    73
<211>    119
<212>    DNA
<213>    Artificial Sequence

<220>
<223>    F5_B_2-2


<400>    73
ttctaaagct aaagaatact tcttttaaat ttcagttttt cttgatcatg aaaatgccaa        60

agaggtacaa tcaggtaaac tggaagagtt tgtttcaggg aaccttgaga gagaatgta        119

<210>    74
<211>    140
<212>    DNA
<213>    Artificial Sequence

<220>
<223>    F5_B_3-1


<400>    74
ttctaaagct aaagaatact tcttttaaat ttcagttttt cttgatcatg aaaatgccac        60

caaaattctg aatcgggcaa agaggtacaa ttcaggtaaa ctggaagagt ttgtttcagg       120

gaaccttgag agagaatgta                                                   140


<210>    75
<211>    161
<212>    DNA
<213>    Artificial Sequence

<220>
<223>    F5_B_3-2


<400>    75
ttctaaagct aaagaatact tcttttaaat ttcagttttt cttgatcatg aaaatgccac        60

caaaattctg aatcgggcaa agaggtacac tggaaagagg gcaaagaggt acactggtaa       120

actggaagag tttgtttcag ggaaccttga gagagaatgt a                           161


<210>    76
<211>    140
<212>    DNA
<213>    Artificial Sequence

<220>
<223>    F5_B_4-1


<400>    76
ttctaaagct aaagaatact tcttttaaat ttcagttttt cttgatcatg aaaatgccac        60

caaaattctg aatcgggcaa agaggtacaa ttcaggtaaa ctggaagagt ttgtttcagg       120

gaaccttgag agagaatgta                                                   140


<210>    77
<211>    135
<212>    DNA
<213>    Artificial Sequence

<220>
<223>    F5_B_4-2


<400>    77

ttctaaagct aaagaatact tcttttaaat ttcagttttt cttgatcatg aaaatgccac 60

caaaattctg gcaaagaggt acaaattcag gtaaactgga agagtttgtt tcagggaacc 120

ttgagagaga atgta 135

<210> 78
<211> 140
<212> DNA
<213> Artificial Sequence

<220>
<223> F5_B_5-1

<400> 78
ttctaaagct aaagaatact tcttttaaat ttcagttttt cttgatcatg aaaatgccac 60

caaaattctg aatcgggcaa agaggtacaa ttcaggtaaa ctggaagagt ttgtttcagg 120

gaaccttgag agagaatgta 140

<210> 79
<211> 155
<212> DNA
<213> Artificial Sequence

<220>
<223> F5_B_5-2

<400> 79
ttctaaagct aaagaatact tcttttaaat ttcagttttt cttgatcatg aaaatgccac 60

caaaattctg gcaaagaggt acactggaaa gagggcaaag aggtacactg gtaaactgga 120

agagtttgtt tcagggaacc ttgagagaga atgta 155

<210> 80
<211> 140
<212> DNA
<213> Artificial Sequence

<220>
<223> F5_B_6-1

<400> 80
ttctaaagct aaagaatact tcttttaaat ttcagttttt cttgatcatg aaaatgccac 60

caaaattctg aatcgggcaa agaggtacaa ttcaggtaaa ctggaagagt ttgtttcagg 120

gaaccttgag agagaatgta 140

<210> 81
<211> 121
<212> DNA
<213> Artificial Sequence

<220>
<223>    F5_B_6-2


<400>    81
ttctaaagct aaagaatact tcttttaaat ttcagttttt cttgatcatg aaaatgccaa        60

agaggtacaa attcaggtaa actggaagag tttgtttcag ggaaccttga gagagaatgt       120

a                                                                       121


<210>    82
<211>    140
<212>    DNA
<213>    Artificial Sequence

<220>
<223>    F5_B_7-1


<400>    82
ttctaaagct aaagaatact tcttttaaat ttcagttttt cttgatcatg aaaatgccac        60

caaaattctg aatcgggcaa agaggtacaa ttcaggtaaa ctggaagagt ttgtttcagg       120

gaaccttgag agagaatgta                                                   140


<210>    83
<211>    133
<212>    DNA
<213>    Artificial Sequence

<220>
<223>    F5_B_7-2


<400>    83
ttctaaagct aaagaatact tcttttaaat ttcagttttt cttgatcatg aaaatgccac        60

caaaattctg gcaaagaggt acaatcaggt aaactggaag agtttgtttc agggaacctt       120

gagagagaat gta                                                          133


<210>    84
<211>    140
<212>    DNA
<213>    Artificial Sequence

<220>
<223>    F5_B_8-1


<400>    84
ttctaaagct aaagaatact tcttttaaat ttcagttttt cttgatcatg aaaatgccac        60

caaaattctg aatcgggcaa agaggtacaa ttcaggtaaa ctggaagagt ttgtttcagg       120

gaaccttgag agagaatgta                                                   140

<210>    85
<211>    117
<212>    DNA
<213>    Artificial Sequence

<220>
<223>    F5_B_8-2


<400>    85
ttctaaagct aaagaatact tcttttaaat ttcagttttt cttgatcatg aaaatgccaa        60

agaggtacac tggtaaactg gaagagtttg tttcagggaa ccttgagaga gaatgta          117


<210>    86
<211>    140
<212>    DNA
<213>    Artificial Sequence

<220>
<223>    F6_A_1-1


<400>    86
tcttttaaat ttcagttttt cttgatcatg aaaatgccac caaaattctg aatcgggcaa        60

agaggtacaa ttcaggtaaa ctggaagagt ttgtttcagg gaaccttgag agagaatgta       120

tagaagaaag gtgtagtttt                                                   140


<210>    87
<211>    80
<212>    DNA
<213>    Artificial Sequence

<220>
<223>    F6_A_1-2


<400>    87
tcttttaaat ttcaggtaaa ctggaagagt ttgtttcagg gaaccttgag agagaatgta        60

tagaagaaag gtgtagtttt                                                    80


<210>    88
<211>    140
<212>    DNA
<213>    Artificial Sequence

<220>
<223>    F6_A_2-1


<400>    88
tcttttaaat ttcagttttt cttgatcatg aaaatgccac caaaattctg aatcgggcaa        60

agaggtacaa ttcaggtaaa ctggaagagt ttgtttcagg gaaccttgag agagaatgta       120

tagaagaaag gtgtagtttt                                                   140

<210>	89
<211>	129
<212>	DNA
<213>	Artificial Sequence

<220>
<223>	F6_A_2-2


<400>	89
tcttttaaat ttcagttttt cttgatcatg aaaatgccac caaaattctg aatcgggcaa	60

agaggtaaac tggaagagtt tgtttcaggg aaccttgaga gagaatgtat agaagaaagg	120

tgtagtttt	129


<210>	90
<211>	140
<212>	DNA
<213>	Artificial Sequence

<220>
<223>	F6_A_3-1


<400>	90
tcttttaaat ttcagttttt cttgatcatg aaaatgccac caaaattctg aatcgggcaa	60

agaggtacaa ttcaggtaaa ctggaagagt ttgtttcagg gaaccttgag agagaatgta	120

tagaagaaag gtgtagtttt	140


<210>	91
<211>	123
<212>	DNA
<213>	Artificial Sequence

<220>
<223>	F6_A_3-2


<400>	91
tcttttaaat ttcagttttt cttgatcatg aaaatgccac caaaattcgg gcaaagaggt	60

aaactggaag agtttgtttc agggaacctt gagagagaat gtatagaaga aaggtgtagt	120

ttt	123


<210>	92
<211>	140
<212>	DNA
<213>	Artificial Sequence

<220>
<223>	F6_B_1-1


<400>	92
tcttttaaat ttcagttttt cttgatcatg aaaatgccac caaaattctg aatcgggcaa	60

agaggtacaa ttcaggtaaa ctggaagagt ttgtttcagg gaaccttgag agagaatgta 120

tagaagaaag gtgtagtttt 140

<210> 93
<211> 103
<212> DNA
<213> Artificial Sequence

<220>
<223> F6_B_1-2

<400> 93
tcttttaaat ttcagttttt cttgatcatg aaaatgccac caactggaag agtttgtttc 60

agggaacctt gagagagaat gtatagaaga aaggtgtagt ttt 103

<210> 94
<211> 140
<212> DNA
<213> Artificial Sequence

<220>
<223> F6_B_2-1

<400> 94
tcttttaaat ttcagttttt cttgatcatg aaaatgccac caaaattctg aatcgggcaa 60

agaggtacaa ttcaggtaaa ctggaagagt ttgtttcagg gaaccttgag agagaatgta 120

tagaagaaag gtgtagtttt 140

<210> 95
<211> 132
<212> DNA
<213> Artificial Sequence

<220>
<223> F6_B_2-2

<400> 95
tcttttaaat ttcagttttt cttgatcatg aaaatgccac caaaattctg ggcaaagagg 60

taaacaggta aactggaaga gtttgtttca gggaaccttg agagagaatg tatagaagaa 120

aggtgtagtt tt 132

<210> 96
<211> 140
<212> DNA
<213> Artificial Sequence

<220>
<223> F6_B_3-1

<400>    96
tcttttaaat ttcagttttt cttgatcatg aaaatgccac caaaattctg aatcgggcaa      60

agaggtacaa ttcaggtaaa ctggaagagt ttgtttcagg gaaccttgag agagaatgta     120

tagaagaaag gtgtagtttt                                                 140


<210>    97
<211>    129
<212>    DNA
<213>    Artificial Sequence

<220>
<223>    F6_B_3-2


<400>    97
tcttttaaat ttcagttttt cttgatcatg aaaatgccac caaaattctg aatcgggcaa      60

agaggtaaac tggaagagtt tgtttcaggg aaccttgaga gagaatgtat agaagaaagg     120

tgtagtttt                                                             129


<210>    98
<211>    140
<212>    DNA
<213>    Artificial Sequence

<220>
<223>    F6_B_4-1


<400>    98
tcttttaaat ttcagttttt cttgatcatg aaaatgccac caaaattctg aatcgggcaa      60

agaggtacaa ttcaggtaaa ctggaagagt ttgtttcagg gaaccttgag agagaatgta     120

tagaagaaag gtgtagtttt                                                 140


<210>    99
<211>    124
<212>    DNA
<213>    Artificial Sequence

<220>
<223>    F6_B_4-2


<400>    99
tcttttaaat ttcagttttt cttgatcatg aaaatgccac caaaattctg ggcaaagagg      60

taaactggaa gagtttgttt cagggaacct tgagagagaa tgtatagaag aaaggtgtag     120

tttt                                                                  124


<210>    100
<211>    140
<212>    DNA

<213>    Artificial Sequence

<220>
<223>    F6_B_5-1


<400>    100
tcttttaaat ttcagttttt cttgatcatg aaaatgccac caaaattctg aatcgggcaa        60

agaggtacaa ttcaggtaaa ctggaagagt ttgtttcagg gaaccttgag agagaatgta       120

tagaagaaag gtgtagtttt                                                   140


<210>    101
<211>    137
<212>    DNA
<213>    Artificial Sequence

<220>
<223>    F6_B_5-2


<400>    101
tcttttaaat ttcagttttt cttgatcatg aaaatgccac caaaattctg aatcgggcaa        60

agaggtaaac aggtaaactg gaagagtttg tttcagggaa ccttgagaga gaatgtatag       120

aagaaaggtg tagtttt                                                      137


<210>    102
<211>    140
<212>    DNA
<213>    Artificial Sequence

<220>
<223>    F6_B_6-1


<400>    102
tcttttaaat ttcagttttt cttgatcatg aaaatgccac caaaattctg aatcgggcaa        60

agaggtacaa ttcaggtaaa ctggaagagt ttgtttcagg gaaccttgag agagaatgta       120

tagaagaaag gtgtagtttt                                                   140


<210>    103
<211>    103
<212>    DNA
<213>    Artificial Sequence

<220>
<223>    F6_B_6-2


<400>    103
tcttttaaat ttcagttttt cttgatcatg aaaatgccac caactggaag agtttgtttc        60

agggaacctt gagagagaat gtaaagaaga aaggtgtagt ttt                         103

<210>    104
<211>    140
<212>    DNA
<213>    Artificial Sequence

<220>
<223>    F6_B_7-1


<400>    104
tcttttaaat ttcagttttt cttgatcatg aaaatgccac caaaattctg aatcgggcaa    60

agaggtacaa ttcaggtaaa ctggaagagt ttgtttcagg gaaccttgag agagaatgta    120

tagaagaaag gtgtagtttt                                                140


<210>    105
<211>    137
<212>    DNA
<213>    Artificial Sequence

<220>
<223>    F6_B_7-2


<400>    105
tcttttaaat ttcagttttt cttgatcatg aaaatgccac caaaattctg ccaccaaaaa    60

tcgggcaaag aggtaaactg gaagagtttg tttcagggaa ccttgagaga gaatgtatag    120

aagaaaggtg tagtttt                                                   137


<210>    106
<211>    140
<212>    DNA
<213>    Artificial Sequence

<220>
<223>    F6_B_8-1


<400>    106
tcttttaaat ttcagttttt cttgatcatg aaaatgccac caaaattctg aatcgggcaa    60

agaggtacaa ttcaggtaaa ctggaagagt ttgtttcagg gaaccttgag agagaatgta    120

tagaagaaag gtgtagtttt                                                140


<210>    107
<211>    132
<212>    DNA
<213>    Artificial Sequence

<220>
<223>    F6_B_8-2


<400>    107
tcttttaaat ttcagttttt cttgatcatg aaaatgccac caaaattctg ggcaaagagg    60

taaacaggta aactggaaga gtttgtttca gggaaccttg agagagaatg taaagaagaa 120

aggtgtagtt tt 132

<210> 108
<211> 140
<212> DNA
<213> Artificial Sequence

<220>
<223> F6_B_9-1

<400> 108
tcttttaaat ttcagttttt cttgatcatg aaaatgccac caaaattctg aatcgggcaa 60

agaggtacaa ttcaggtaaa ctggaagagt ttgtttcagg gaaccttgag agagaatgta 120

tagaagaaag gtgtagtttt 140

<210> 109
<211> 129
<212> DNA
<213> Artificial Sequence

<220>
<223> F6_B_9-2

<400> 109
tcttttaaat ttcagttttt cttgatcatg aaaatgccac caaaattctg aatcgggcaa 60

agaggtaaac tggaagagtt tgtttcaggg aaccttgaga gagaatgtaa agaagaaagg 120

tgtagtttt 129

<210> 110
<211> 140
<212> DNA
<213> Artificial Sequence

<220>
<223> F6_B_10-1

<400> 110
tcttttaaat ttcagttttt cttgatcatg aaaatgccac caaaattctg aatcgggcaa 60

agaggtacaa ttcaggtaaa ctggaagagt ttgtttcagg gaaccttgag agagaatgta 120

tagaagaaag gtgtagtttt 140

<210> 111
<211> 123
<212> DNA
<213> Artificial Sequence

<220>
<223> F6_B_10-2

<400>    111
tcttttaaat ttcagttttt cttgatcatg aaaatgccac caaaaatcgg gcaaagaggt        60

aaactggaag agtttgtttc agggaacctt gagagagaat gtatagaaga aaggtgtagt       120

ttt                                                                     123


<210>    112
<211>    140
<212>    DNA
<213>    Artificial Sequence

<220>
<223>    F6_C_1-1


<400>    112
atgatgtttt cttttttcta aagctaaaga atacttcttt taaatttcag tttttcttga        60

tcatgaaaat gccaccaaaa ttctgaactg tgagtatttc cacataataa cttttttttt       120

tttattttga caggtagagt                                                   140


<210>    113
<211>    111
<212>    DNA
<213>    Artificial Sequence

<220>
<223>    F6_C_1-2


<400>    113
atgatgtttt cttttttcta aagctaaaga atacttcttt taaatttcag tttttcttga        60

tcatgaaaat gccaccaaaa ttctgaactg tgagtatttc cacataataa c                111


<210>    114
<211>    140
<212>    DNA
<213>    Artificial Sequence

<220>
<223>    F6_C_2-1


<400>    114
atgatgtttt cttttttcta aagctaaaga atacttcttt taaatttcag tttttcttga        60

tcatgaaaat gccaccaaaa ttctgaactg tgagtatttc cacataataa cttttttttt       120

tttattttga caggtagagt                                                   140


<210>    115
<211>    137
<212>    DNA
<213>    Artificial Sequence

<220>
<223>    F6_C_2-2


<400>    115
   atgatgtttt cttttttcta aagctaaaga atacttcttt taaatttcag tttttcttga         60
   tcatgaaaat gccaccaaaa ttctgaactg tgagtatttc cacataataa cttttttttt        120
   attttgacag gtagagt                                                       137


<210>    116
<211>    140
<212>    DNA
<213>    Artificial Sequence

<220>
<223>    F6_C_3-1


<400>    116
   atgatgtttt cttttttcta aagctaaaga atacttcttt taaatttcag tttttcttga         60
   tcatgaaaat gccaccaaaa ttctgaactg tgagtatttc cacataataa cttttttttt        120
   tttattttga caggtagagt                                                    140


<210>    117
<211>    139
<212>    DNA
<213>    Artificial Sequence

<220>
<223>    F6_C_3-2


<400>    117
   atgatgtttt cttttttcta aagctaaaga atacttcttt taaatttcag tttttcttga         60
   tcatgaaaat gccaccaaaa ttctgaactg tgagtatttc cacataataa cttttttttt        120
   ttattttgac aggtagagt                                                     139


<210>    118
<211>    140
<212>    DNA
<213>    Artificial Sequence

<220>
<223>    F6_C_4-1


<400>    118
   atgatgtttt cttttttcta aagctaaaga atacttcttt taaatttcag tttttcttga         60
   tcatgaaaat gccaccaaaa ttctgaactg tgagtatttc cacataataa cttttttttt        120
   tttattttga caggtagagt                                                    140

<210>    119
<211>    138
<212>    DNA
<213>    Artificial Sequence

<220>
<223>    F6_C_4-2


<400>    119
   atgatgtttt cttttttcta aagctaaaga atacttcttt taaatttcag tttttcttga      60
   tcatgaaaat gccaccaaaa ttctgaactg tgagtatttc cacataataa cttttttttt      120
   tattttgaca ggtagagt                                                    138


<210>    120
<211>    140
<212>    DNA
<213>    Artificial Sequence

<220>
<223>    F6_C_5-1


<400>    120
   atgatgtttt cttttttcta aagctaaaga atacttcttt taaatttcag tttttcttga      60
   tcatgaaaat gccaccaaaa ttctgaactg tgagtatttc cacataataa cttttttttt      120
   tttattttga caggtagagt                                                  140


<210>    121
<211>    136
<212>    DNA
<213>    Artificial Sequence

<220>
<223>    F6_C_5-2


<400>    121
   atgatgtttt cttttttcta aagctaaaga atacttcttt taaatttcag tttttcttga      60
   tcatgaaaat gccaccaaaa ttctgaactg tgagtatttc cacataataa ctttttttta      120
   ttttgacagg tagagt                                                      136


<210>    122
<211>    74
<212>    DNA
<213>    Artificial Sequence

<220>
<223>    F9_1-1


<400>    122
ctttagtgcc accagaagat actacctggg tgcagtggaa ctgtcctggg actatatgca      60

cagtgacctg ctca                                                    74

<210>    123
<211>    70
<212>    DNA
<213>    Artificial Sequence

<220>
<223>    F9_1-2

<400>    123
ctttagtgcc accagaagat actgggtgca gtggaactgt cctgggacta tatgcacagt    60

gacctgctca                                                          70

<210>    124
<211>    74
<212>    DNA
<213>    Artificial Sequence

<220>
<223>    F9_5-1

<400>    124
ctttagtgcc accagaagat actacctggg tgcagtggaa ctgtcctggg actatatgca    60

cagtgacctg ctca                                                    74

<210>    125
<211>    70
<212>    DNA
<213>    Artificial Sequence

<220>
<223>    F9_5-2

<400>    125
ctttagtgcc accagaagat actgggtgca gtggaactgt cctgggacta tatgcacagt    60

gacctgctca                                                          70

<210>    126
<211>    61
<212>    DNA
<213>    Artificial Sequence

<220>
<223>    F10_4-1

<400>    126
tcatgaaaat gccaccaaaa ttctgaatcg gcaaagaggg tacaattcag gtaaactgga    60

a                                                                   61

<210>	127
<211>	56
<212>	DNA
<213>	Artificial Sequence

<220>
<223>	F10_4-2


<400>	127
tcatgaaaat gccaccaaaa ttcgggcaaa gaggtacaaa ttcaggtaaa ctggaa	56


<210>	128
<211>	61
<212>	DNA
<213>	Artificial Sequence

<220>
<223>	F10_4-3


<400>	128
tcatgaaaat gccaccaaaa ttctgaatcg ggcaaagagg tacaattcag gtaaactgga	60

a	61


<210>	129
<211>	55
<212>	DNA
<213>	Artificial Sequence

<220>
<223>	F10_4-4


<400>	129
tcatgaaaat gccaccaaaa ttcgggcaaa gaggtacaat tcaggtaaac tggaa	55


<210>	130
<211>	61
<212>	DNA
<213>	Artificial Sequence

<220>
<223>	F10_4-5


<400>	130
tcatgaaaat gccaccaaaa ttctgaatcg ggcaaagagg tacaattcag gtaaactgga	60

a	61


<210>	131
<211>	62
<212>	DNA
<213>	Artificial Sequence

<220>

<223>    F10_4-6

<400>    131
tcatgaaaat gccaccaaaa ttctgaatcg ggcaaagagg tacaaattca ggtaaactgg    60

aa                                                                  62

<210>    132
<211>    76
<212>    DNA
<213>    Artificial Sequence

<220>
<223>    F11_1-4-1

<400>    132
gagctttagt gccaccagaa gatactgggt gcagtggaac tgtcctggga ctatatgcac    60

agtgacctgc tcagtg                                                   76

<210>    133
<211>    75
<212>    DNA
<213>    Artificial Sequence

<220>
<223>    F11_1-4-2

<400>    133
gagctttagt gccaccagaa gatactgggt gcagtggaac tgtcctgggc tatatgcaca    60

gtgacctgct cagtg                                                    75

<210>    134
<211>    74
<212>    DNA
<213>    Artificial Sequence

<220>
<223>    F11_5-1-1

<400>    134
ctttagtgcc accagaagat actacctggg tgcagtggaa ctgtcctggg actatatgca    60

cagtgacctg ctca                                                     74

<210>    135
<211>    70
<212>    DNA
<213>    Artificial Sequence

<220>
<223>    F11_5-1-2

<400>    135
ctttagtgcc accagaagat actgggtgca gtggaactgt cctgggacta tatgcacagt        60

gacctgctca                                                               70



<210>    136
<211>    59
<212>    DNA
<213>    Artificial Sequence

<220>
<223>    F12_4-1-1



<400>    136
ttgatcatga aaatgccacc aaaattcggg caaagaggta caaattcagg taaactgga         59



<210>    137
<211>    58
<212>    DNA
<213>    Artificial Sequence

<220>
<223>    F12_4-1-2



<400>    137
ttgatcatga aaatgccacc aaattcgggc aaagaggtac aaattcaggt aaactgga          58